# EUROPEAN PATENT SPECIFICATION

(11) **EP 1 701 949 B1**
(45) Date of publication and mention of the grant of the patent: **18.04.2018**
(21) Application number: 04813878.8
(22) Date of filing: 13.12.2004
(51) Int. Cl.: C07D 317/48, C07D 317/58, C07D 405/12, C07K 16/44, G01N 33/532, G01N 33/94, A61K 39/385

(54) **ASSAY FOR ENTACTOGENS**
ASSAY FÜR ENTAKTOGENE
ESSAI D'ENTACTOGENES

(30) Priority: 15.12.2003 US 736004
(43) Date of publication of application: 20.09.2006
(73) Proprietor: Siemens Healthcare Diagnostics Inc., Deerfield, IL 60015 (US)
(72) Inventor: ZHENG, Yi, Feng, Wilmington, DE 19808 (US); LIU, Hshiou-Ting, Milpitas, CA 95035 (US); YANG, Yali, Bear, Delaware 19701 (US)
(74) Representative: Schweitzer, Klaus
(86) International application number: PCT/US2004/041622
(87) International publication number: WO 2005/058865

(56) References cited:
- EP-A- 1 331 219
- EP-A- 1 340 981
- EP-A1- 1 321 772
- WO-A-01/81424
- WO-A-03/061595
- US-A1- 2003 175 995
- US-A1- 2003 207 469
- US-A1- 2005 014 210
- ZHAO H ET AL: "PROFILES OF URINE SAMPLES TAKEN FROM ECSTASY USERS AT RAVE PARTIES: ANALYSIS BY IMMUNOASSAYS, HPLC, AND GC-MS" JOURNAL OF ANALYTICAL TOXICOLOGY, XX, XX, vol. 25, no. 4, May 2001 (2001-05), pages 258-269, XP001070686
- STAACK R F ET AL: "New designer drug p-methoxymethamphetamine: studies on its metabolism and toxicological detection in urine using gas chromatography-mass spectrometry" JOURNAL OF CHROMATOGRAPHY B: BIOMEDICAL SCIENCES & APPLICATIONS, ELSEVIER, AMSTERDAM, NL, vol. 789, no. 1, 5 June 2003 (2003-06-05), pages 27-41, XP004421813 ISSN: 1570-0232
- G.W. KABALKA ET AL.: "selected reductions of conjugated nitroalkenes." TETRAHEDRON., vol. 46, no. 21, 1990, pages 7443-7457, XP002464072 NLELSEVIER SCIENCE PUBLISHERS, AMSTERDAM.
- J.G. CANNON: "n-alkyl derivatives of (+-)-alfa-methyldopamine." JOURNAL OF MEDICINAL CHEMISTRY., vol. 22, no. 8, 1979, pages 901-907, XP002464073 USAMERICAN CHEMICAL SOCIETY. WASHINGTON.
- M.Y. YOUSIF ET AL.: "identifications of metabolites of 3,4-methylenedioxymethamphetamine" DRUG AND ALCOHOL DEPENDENCE, vol. 26, 1990, pages 127-135, XP002464074 ireland

## Description

### BACKGROUND OF THE INVENTION

This invention relates to methods, compositions and kits for detecting the presence and/or amounts of entactogens in samples suspected of containing the same. In particular, the invention relates to haptens, immunogens and assays for 3,4-methylenedioxyamphetamine (MDA), 3,4-methylenedioxy-methamphetamine (MDMA), 3,4-methylenedioxyethamphetamine (MDEA) and 4-hydroxy-3-methoxymethamphetamine (HMMA).

The clinical diagnostic field has seen a broad expansion in recent years, both as to the variety of materials of interest that may be readily and accurately determined, as well as the methods for the determination. Over the last decade, testing for drugs of abuse has become commonplace. This testing is not only for the monitoring of criminal offenders and drug addicts, but employers also use it for the screening of workers. In recent years, immunoassay based on the reaction of an antibody with an antigen has been extensively investigated for this purpose. Immunoassay may be roughly classified into radioimmunoassay, using a radioactive isotope, enzyme-immunoassay (EIA) using an enzyme and luminescence assays, using fluorescent labels, e.g., fluorescence polarization, and chemiluminescent labels.

Amphetamine and methamphetamine stimulate the central nervous system and have been used medicinally to treat hypotension, narcolepsy and obesity. Because of their stimulating effects, the drugs and derivatives have been abused.

The designer drugs, methylenedioxyamphetamine (MDA), 1-3',4'-methylenedioxyphenyl)-2-propanamine, "Love Pills", methylenedioxy-methamphetamine (MDMA), "Adam", "Ecstasy" and methylenedioxyethamphetamine (MDEA), "Eve" are entactogens, producing feeling of euphoria and friendliness. These drugs are currently popular and called "rave drugs". It has been demonstrated by several experimental studies on rats and human that these drugs are risky to human. In fact, toxicity and deaths associated with MDMA has been reported.

Recent reviews have also reported the hepatotoxicity, neurotoxicity, psychopathology and the abuse potential of these drugs. The common use of these drugs has been widespread in the world and appeared recently as the most popular drug of abuse in certain countries.

Although there is a need for the detection of MDMA, MDA and its metabolites such as 4-hydroxy-3-methoxymethamphetamine (HMMA) and so forth, the literature discloses GC-MS, HPLC detection methods, which are expensive and time consuming. It appears that researchers have tried to use existing amphetamine/methamphetamine immunoassay technology for the detection of MDMA and MDA due to their cross-reactivity. The hope was that the antibody recognizing amphetamine and methamphetamine would also be useful for assays for MDMA, MDA or its metabolites. For instance, three commercial amphetamine/methamphetamine assays, namely, EMIT®, FPIA and RIA, have been investigated for the detection of MDA, MDMA and MDEA. (Ruangyuttikarn, et al., "Comparison of three commercial amphetamine immunoassay for detection of methamphetamine, methylenedioxyamphetamine, methylenedioxy-methamphetamine and methylenedioxyethylamphetamine" J. Anal. Toxicol. 1988, 12, 229; Kunsman, et al., "Application of the Syva Emit and Abbott TDX amphetamine Immunoassays to the detection of 3,4-Methylenedioxy-methamphetamine (MDMA) and 3,4-Methylenedioxyethamphetamine (MDEA) in Urine" J. Anal. Toxicol. 1990, 14, 149; Cody, J. T. "Detection of D, L-amphetamine, D,L-methamphetamine, and illicit amphetamine analogs using diagnostic products corporation's amphetamine and methamphetamine radioimmunoassay" J. Anal. Toxicol. 1990, 14, 321; Ensslin, et al., "Toxicological detection of the designer drug 3,4-methylenedioxyethylamphetamine (MDE, 'Eve') and its metabolites in urine by gas chromatography spectrometry and fluorescence polarization immunoassay" J. Chromatogr. 1996, B683, 189.

EP-A 1 340 981 discloses an N-ethylamphetamine derivative as an analyte and immunogen for the production of antibodies specific for an ecstasy drug (preferably MDA (3,4-methylenedioxyamphetamine), MDMA (3,4-methylenedioxy-N-methylamphetamine), MDEA (3,4-methylenedioxy-N-ethylamphetamine), MDPA (3,4-methylenedioxy-N-propylamphetamine), BDB (3,4-methylenedioxyphenyl-2-butanamine) or MBDB (3,4-methylenedioxyphenyl-N-methylbutanamine). It also discloses an antibody specific for an ecstasy drug; a reaction kit comprising the antibody; the preparation of an antibody involving inoculating a host with said immunogen and detecting an analyte in a sample involving contacting the sample with the antibody, binding the antibody to the analyte and detecting an adduct formed by the antibody and the analyte.

EP 1 331 219 A1 discloses compositions based on amphetamine and amphetamine derivatives for the treatment of amphetamine abuse. The compositions include amphetamine derivatives as well as immunoglobulins or fragments or chains thereof which specifically bind to at least two analogs of amphetamine. For polyclonal or monoclonal antibody production, animals are immunized with antigens comprising linking groups bonded to the aromatic group or to a side chain of the amphetamine derivative for conjugation to a carrier protein. The polyclonal or monoclonal antibodies may be used in detection assays for amphetamine and its analogs or for treatment of an overdose of an amphetamine.

However, according to the published literature, the above approaches have achieved little, if any, success. This result is not unexpected due to the very different chemical structures between methamphetamine and MDMA analogs. That is,
MDMA and MDA have an extra (methylenedioxy) five-member ring in comparison to methamphetamine and amphetamine, respectively.

There is, therefore, a need for assays for the detection of the aforementioned designer drugs and, in some instances, their major metabolites. The assays should be able to detect the designer drugs in order to monitor and treat patients addicted to these drugs.

### SUMMARY OF THE INVENTION

One embodiment of the present invention is a compound of the formula: 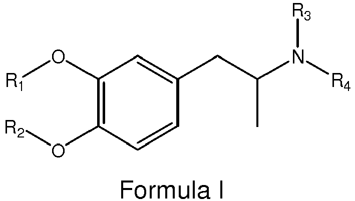 wherein the substituents R₁, R₂, R₃ and R₄ have the meanings as defined in the claims.

Another embodiment of the present invention is a method for determining a compound selected from the group consisting of 3,4-methylenedioxyamphetamine (MDA), 3,4-methylenedioxy-methamphetamine (MDMA), 3,4-methylenedioxy-ethylamphetamine (MDEA) and 4-hydroxy-3-methoxy-methamphetamine (HMMA). The method comprises providing in combination in a medium (i) a sample suspected of containing the compound and (ii) an antibody raised against a compound of the formula shown in claim 3, step a) that comprises a protein. The medium is examined for the presence a complex comprising the compound and the antibody where the presence of such as complex indicates the presence of the compound in the sample. In one aspect of the above embodiment, the combination further comprises a label conjugate of the above compound.

Another embodiment of the present invention is a kit for determining a compound selected from the group consisting of 3,4-methylenedioxyamphetamine (MDA), 3,4-methylenedioxy-methamphetamine (MDMA), 3,4-methylenedioxy-ethylamphetamine (MDEA) and 4-hydroxy-3-methoxy-methamphetamine (HMMA). The kit comprises (a) an antibody raised against said compound that comprises a protein and (c) ancillary reagents for determining the compound. The kit further comprises (b) a label conjugate of the compound of the above formula.

### BRIEF DESCRIPTION OF THE DRAWINGS

Figure 5 is a reaction scheme depicting an example of a synthesis of HMMA intermediate (29) for immunogen (30).
Figure 6 is a reaction scheme depicting an example of a synthesis of HMMA-KLH immunogen (30).
Figure 7 is a reaction scheme depicting an example of a synthesis of HMMA-KLH immunogen (36).
Figure 8A is a reaction scheme depicting an example of a synthesis of MDMA hapten (38).
Figure 8B is a reaction scheme depicting an example of a synthesis of MDMA hapten intermediate (40).

### DESCRIPTION OF THE SPECIFIC EMBODIMENTS

Immunogens comprising proteins are synthesized and used to prepare antibodies specific for 3,4-methylenedioxyamphetamine (MDA), 3,4-methylenedioxy-methamphetamine (MDMA), 3,4-methylenedioxy-ethylamphetamine (MDEA) and 4-hydroxy-3-methoxymethamphetamine (HMMA). The antibodies may be used in methods for detecting the aforementioned drugs in samples suspected of containing the drugs. Label conjugates are prepared and may be employed in the above methods. Effective screening of samples for the presence of one or more entactogens as referred to above may be realized.

The immunogens and label conjugates may involve an analog of MDA, MDMA, MDEA or HMMA linked through the nitrogen, or in the case of HMMA alternatively through the 4-position of the benzene ring, to a protein or a label, respectively. The linking group may comprise about 2 to about 10 atoms not counting hydrogen and may comprise a chain of from 2 to 8 atoms, each independently selected from the group normally consisting of carbon, oxygen, sulfur, nitrogen, halogen and phosphorous. Where the linking group provides attachment of a protein to the 4-position of the benzene ring of HMMA, the linking group usually comprises at least 5 atoms or, when less than 5 atoms, the linking group does not comprise solely carbon or oxygen.

The number of heteroatoms in the linking groups will normally range from about 0 to 6, usually from about 1 to 5. The linking groups may be aliphatic or aromatic. When heteroatoms are present, oxygen will normally be present as oxo or oxy, bonded to carbon, sulfur, nitrogen or phosphorous, nitrogen will normally be present as nitro, nitroso or amino, normally bonded to carbon, oxygen, sulfur or phosphorous; sulfur would be analogous to oxygen; while phosphorous will be bonded to carbon, sulfur, oxygen or nitrogen, usually as phosphonate and phosphate mono- or diester. Common functionalities in forming a covalent bond between the linking group and the molecule to be conjugated are alkylamine, amidine, thioamide, ether, urea, thiourea, guanidine, azo, thioether and carboxylate, sulfonate, and phosphate esters, amides and thioesters.

For the most part, when a linking group has a non-oxocarbonyl group including nitrogen and sulfur analogs, a phosphate group, an amino group, alkylating agent such as halo or tosylalkyl, oxy (hydroxyl or the sulfur analog, mercapto) oxocarbonyl (e.g., aldehyde or ketone), or active olefin such as a vinyl sulfone or α-, β-unsaturated ester, these functionalities will be linked to amine groups, carboxyl groups, active olefins, alkylating agents, e.g., bromoacetyl. Where an amine and carboxylic acid or its nitrogen derivative or phosphoric acid is linked, amides, amidines and phosphoramides will be formed. Where mercaptan and activated olefin are linked, thioethers will be formed. Where a mercaptan and an alkylating agent are linked, thioethers will be formed. Where aldehyde and an amine are linked under reducing conditions, an alkylamine will be formed. Where a carboxylic acid or phosphate acid and an alcohol are linked, esters will be formed. Various linking groups are well known in the art; see, for example, Cautrecasas, J. Biol. Chem. (1970) 245:3059.

As mentioned above, one aspect concerns compounds of the formula: 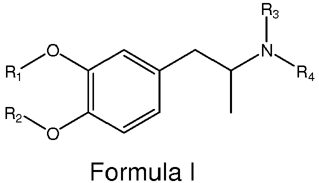 wherein R₁, R₂, R₃ and R₄ have the meaning as defined in the claims.

By the term "lower alkyl" is meant a branched or unbranched saturated monovalent hydrocarbon radical containing 1 to 10, usually, 1 to 5, carbon atoms, such as methyl, ethyl, propyl, butyl and pentyl, and including the normal, secondary, tertiary, and the like, forms thereof where appropriate.
By the term "acid salts thereof" is meant salts formed with acids such as mineral acids, for example, hydrochloric acid, and the like, organic acids, for example, trifluoroacetic acid and the like.

By "-succinimidyl" is meant the following:

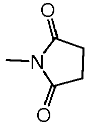

By "-maleimidyl" is meant the following:

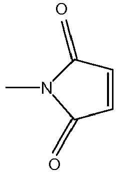

By the term "label" is meant a member of a signal producing system. The label is capable of being detected directly or is detectable through a specific binding reaction that produces a detectable signal. The labels generally are radioisotopic, luminescent, or enzymic. The label can be isotopic or non-isotopic, usually non-isotopic, and can be a catalyst which is an enzyme, a chemiluminescent molecule, coenzyme, enzyme substrate, radioactive group, a small organic molecule, amplifiable polynucleotide sequence, a particle such as latex or carbon particle, metal sol, crystallite, liposome, cell, etc., which may or may not be further labeled with a dye, catalyst or other detectable group, and the like.

The signal producing system may have one or more components, at least one component being the label. The signal producing system generates a signal that relates to the presence of an entactogen in a sample. The signal producing system includes all of the reagents required to produce a measurable signal. Other components of the signal producing system may be included in a developer solution and can include substrates, enhancers, activators, chemiluminescent compounds, cofactors, inhibitors, scavengers, metal ions, specific binding substances required for binding of signal generating substances, and the like. Other components of the signal producing system may be coenzymes, substances that react with enzymic products, other enzymes and catalysts, and the like. The signal producing system provides a signal detectable by external means, by use of electromagnetic radiation, desirably by visual examination. Exemplary signal-producing systems are described in U.S. Patent No. 5,508,178 (Rose, et al.).

By the term "immunogenic carrier" is meant a group which, when conjugated to a hapten and injected into a mammal, will induce an immune response and elicit the production of antibodies that bind to the hapten. Haptens are compounds capable of binding specifically to corresponding antibodies, but do not themselves act as immunogens (or antigens) for preparation of the antibodies. Antibodies that recognize a hapten can be prepared against compounds comprised of the hapten linked to an immunogenic (or antigenic) carrier. Immunogenic carriers are also referred to as antigenic carriers. Typical immunogenic carriers include, without limitation, poly(amino acids), polysaccharides, nucleic acids and particles (biologic and synthetic materials). A wide variety of such carriers are disclosed in Davalian, et al., U.S. Patent No. 5,089,390, column 4, line 57 to column 5, line 5. Immunogenic carriers include proteins such as, for example, albumins, serum proteins, e.g., globulins, ocular lens proteins and lipoproteins, and so forth. Illustrative proteins include bovine serum albumin (BSA), keyhole limpet hemocyanin ("KLH"), egg ovalbumin, bovine gamma-globulin (BGG) and the like.

### Synthesis

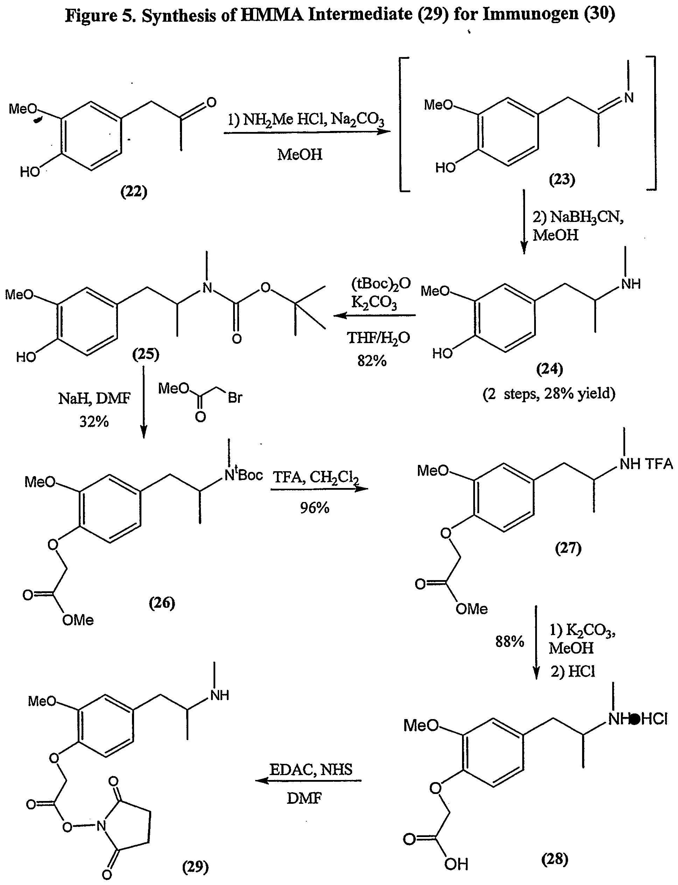

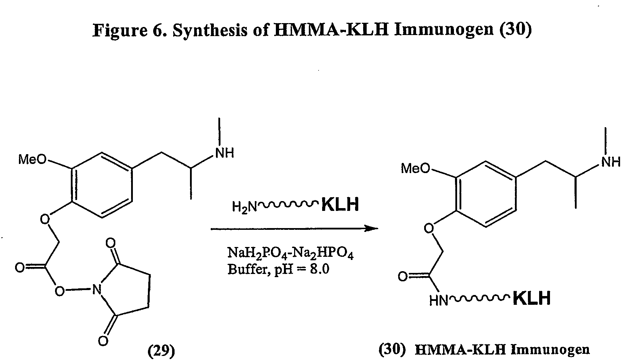

The synthesis of representative examples of the above compounds is discussed herein by way of illustration and not limitation. Other synthetic procedures will be suggested to those skilled in the art in view of the disclosure herein. Other compounds within the scope of the present invention may be prepared using suitable variants of the reagents employed below.
An exemplary synthesis of a HMMA immunogen is depicted in Figure 5 and Figure 6. Referring to Figure 5, Ketone 22 is subjected to reductive amination in a two-step process. In the first step, ketone 22 is reacted, for example, with methylamine hydrochloride under basic conditions. Generally, a buffered aqueous medium containing an organic solvent, for instance, an alcohol, e.g., methanol or the like, is employed. This step yields intermediate compound 23, which is treated with a reducing agent such as a metal hydride, for example, NaBH₃CN in an organic solvent such as an alcohol, e.g., methanol, and the like. The aqueous medium may contain a carbonate buffer such as sodium carbonate, potassium carbonate, and the like. Compound 24 is obtained and treated to protect the amine functionality, thus yielding compound 25. Suitable protecting groups are well known in the art and have been described in detail in numerous patents and articles in the technical literature. See, for example, "Principles of Peptide Synthesis" (M. Bodanszky, Springer Verlag, Berlin, Heidelberg, New York, Tokyo (1984). Examples of such protecting groups, by way of example and not limitation, are t-butoxycarbonyl (t-Boc), fluorenylmethyloxycarbonyl (Fmoc), acetaminomethyl (Acm), triphenyl methyl (Trt), benzyloxycarbonyl, biphenylisopropyloxycarbonyl, 1-amyloxycarbonyl, isobornyloxycarbonyl, alpha-dimethyl-3,5-dimethoxybenzyloxycarbonyl, o-nitrophenyl-sulfenyl, 2-cyano-1,1-dimethyl-ethoxycarbonyl bromobenzyloxy, carbamoyl, formyl, and the like. The particular protecting group chosen depends on the nature of the reaction to be performed and the conditions of such reaction such as temperature, pH, and so forth.
Referring again to Figure 5, the protecting group employed in this exemplary synthesis is di-tert-butyldicarbonate ((tBoc)₂O) in an aqueous organic solvent such as an ether, e.g., THF and the like. A suitable carbonate such as potassium carbonate, sodium carbonate, and the like, is included in the reaction medium to provide for basic conditions. Reaction of 25 with methyl bromoacetate in the presence of a metal hydride, e.g., NaH, in an organic solvent, e.g., DMF, yields compound 26. The protecting group is removed under acidic conditions in an organic solvent to give compound 27. In the example depicted, compound 26 is treated with trifluoroacetic acid (TFA) in methylene chloride. In general, removal of the protecting group is dependent on the nature of the protecting group. Suitable conditions for removal of protecting groups are well known in the art. Compound 27 is treated to convert it to the hydrochloric acid salt compound 28. Exemplary conditions involve hydrolysis under basic conditions such as, e.g., a carbonate such as potassium carbonate in an organic solvent such as methanol. Subsequently, hydrochloric acid is added to form the hydrochloric acid salt. Compound 28 is treated to form activated ester compound 29. Exemplary conditions include, e.g., reacting with EDAC and NHS. Referring to Figure 6, immunogen 30 is obtained by reaction of compound 29 with a protein, e.g., KLH and the like, in a suitable buffer of pH about 7.5 to about 9.0, preferably, about 8, such as, e.g., a phosphate buffer, e.g., sodium phosphate (0.1 M, pH = 8.0) and the like. Immunogen 30 may be purified as discussed above.

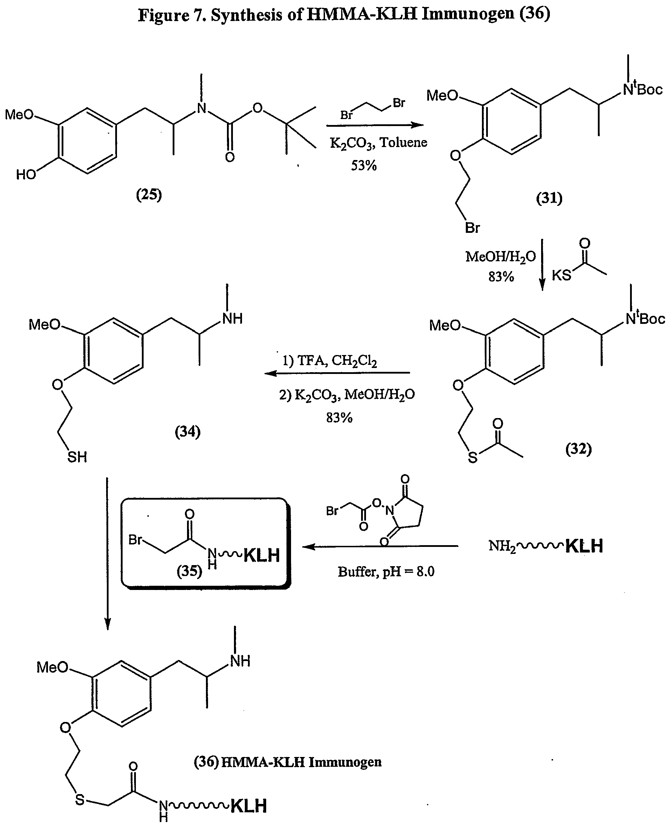

The synthesis of other immunogens of HMMA may be achieved (Figure 7) by using common intermediate compound 25. Alkylation of the phenolic group of compound 25 may be achieved using a suitable di-activated alkane such as, for example, dibromoethane and so forth under basic conditions such as, for example, a carbonate, e.g., potassium carbonate and the like, in an organic solvent such as an aromatic compound, e.g., toluene and the like. The product of the above reaction, namely, compound 31 is treated with a thioester salt of an organic acid such as, for example, potassium thioacetate, potassium thiopropanoate and the like, in an aqueous organic solvent such as methanol water and so forth. The resulting compound 32 is then treated to remove the protecting group and hydrolyze the acetate, in a manner similar to that described above, to obtain thiol compound 34. Bromoacetyl-KLH 35 is obtained by reaction of KLH with an activated ester of bromoacetic acid, namely, the N-hydroxy succinimide ester of bromoacetic acid in this example, under basic conditions, as discussed above for the preparation of compound 15. Reaction of thiol 34 with bromoacetyl-KLH 35 yields immunogenic compound 36, which may be purified as discussed above.

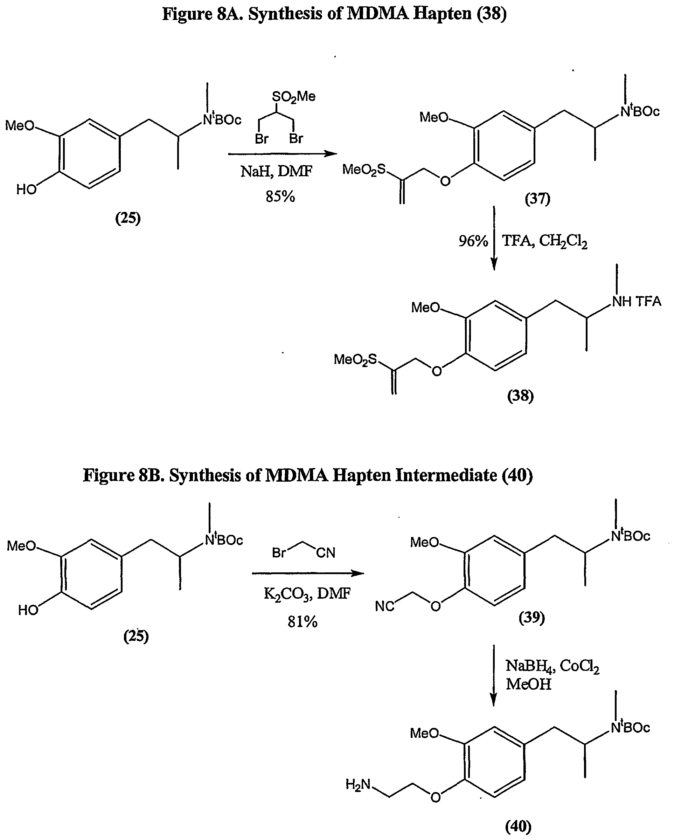

The synthesis of HMMA vinyl sulfone hapten 38 is set forth in Figure 8. Reaction of compound 25 with 1,3-dibromo-2-(methylsulfonyl)propane under basic condition gives compound 37. The reactions are similar to those described above for the syntheses of Figure 4. Hapten 38 is obtained by removal of the protecting group as discussed above.

Referring again to Figure 8, alkylation of phenol 25 with, for example, bromoacetonitrile, under basic conditions such as, e.g., a carbonate in an organic solvent, gives intermediate compound 39. The basic conditions are similar to those described above. In the example depicted, potassium carbonate in DMF is employed at elevated temperature, e.g., about 80°C. Reduction of 39 with a metal hydride such as, e.g., NaBH₄ and the like, in the presence of a Group VIII metal halide (chloride, bromide, fluoride and iodide) such as, for example, CoCl₂, FeCl₂ and so forth, yields amine 40, which may be employed to synthesize a hapten and also an immunogen by reaction with an immunogenic carrier.

The assays of the present invention usually involve reactions between binding partners such as an entactogen analyte and a corresponding antibody or the binding between an antibody and a corresponding binding partner such as a second antibody that binds to the first antibody. Accordingly, the binding partner may be a protein, which may be an antibody or an antigen. The binding partner may be a member of a specific binding pair ("sbp member"), which is one of two different molecules, having an area on the surface or in a cavity, which specifically binds to and is thereby defined as complementary with a particular spatial and polar organization of the other molecule. The members of the specific binding pair will usually be members of an immunological pair such as antigen-antibody, although other specific binding pairs such as biotin-avidin, hormones-hormone receptors, enzyme-substrate, nucleic acid duplexes, IgG-protein A, polynucleotide pairs such as DNA-DNA, DNA-RNA, and the like are not immunological pairs but are included within the scope of sbp member.

Accordingly, specific binding involves the specific recognition of one of two different molecules for the other compared to substantially less recognition of other molecules. On the other hand, non-specific binding involves non-covalent binding between molecules that is relatively independent of specific surface structures. Non-specific binding may result from several factors including hydrophobic interactions between molecules. Preferred binding partners are antibodies.

The immunogens prepared in accordance with the present invention may be employed to prepare antibodies specific for a respective entactogen mentioned above. An antibody is an immunoglobulin that specifically binds to and is thereby defined as complementary with a particular spatial and polar organization of another molecule. The antibody can be monoclonal or polyclonal and can be prepared by techniques that are well known in the art such as immunization of a host and collection of sera (polyclonal) or by preparing continuous hybrid cell lines and collecting the secreted protein (monoclonal) or by cloning and expressing nucleotide sequences or mutagenized versions thereof coding at least for the amino acid sequences required for specific binding of natural antibodies.

Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')₂, Fab', and the like. In addition, aggregates, polymers, and conjugates of immunoglobulins or their fragments can be used where appropriate so long as binding affinity for a particular molecule is maintained.

Antiserum containing antibodies (polyclonal) is obtained by well-established techniques involving immunization of an animal, such as a rabbit, guinea pig, or goat, with an appropriate immunogen and obtaining antisera from the blood of the immunized animal after an appropriate waiting period. State-of-the-art reviews are provided by Parker, Radioimmunoassay of Biologically Active Compounds, Prentice-Hall (Englewood Cliffs, N.J., U.S., 1976), Butler, J. Immunol. Meth. 7: 1-24 (1975); Broughton and Strong, Clin. Chem. 22: 726-732 (1976); and Playfair, et al., Br. Med. Bull. 30: 24-31 (1974).

Antibodies can also be obtained by somatic cell hybridization techniques, such antibodies being commonly referred to as monoclonal antibodies. Monoclonal antibodies may be produced according to the standard techniques of Köhler and Milstein, Nature 265:495-497, 1975. Reviews of monoclonal antibody techniques are found in Lymphocyte Hybridomas, ed. Melchers, et al. Springer-Verlag (New York 1978), Nature 266: 495 (1977), Science 208: 692 (1980), and Methods of Enzymology 73 (Part B): 3-46 (1981). Samples of an appropriate immunogen preparation are injected into an animal such as a mouse and, after a sufficient time, the animal is sacrificed and spleen cells obtained. Alternatively, the spleen cells of a non-immunized animal can be sensitized to the immunogen in vitro. The spleen cell chromosomes encoding the base sequences for the desired immunoglobulins can be compressed by fusing the spleen cells, generally in the presence of a non-ionic detergent, for example, polyethylene glycol, with a myeloma cell line. The resulting cells, which include fused hybridomas, are allowed to grow in a selective medium, such as HAT-medium, and the surviving immortalized cells are grown in such medium using limiting dilution conditions. The cells are grown in a suitable container, e.g., microtiter wells, and the supernatant is screened for monoclonal antibodies having the desired specificity.

Various techniques exist for enhancing yields of monoclonal antibodies, such as injection of the hybridoma cells into the peritoneal cavity of a mammalian host, which accepts the cells, and harvesting the ascites fluid. Where an insufficient amount of the monoclonal antibody collects in the ascites fluid, the antibody is harvested from the blood of the host. Alternatively, the cell producing the desired antibody can be grown in a hollow fiber cell culture device or a spinner flask device, both of which are well known in the art. Various conventional ways exist for isolation and purification of the monoclonal antibodies from other proteins and other contaminants (see Köhler and Milstein, supra).

In another approach for the preparation of antibodies the sequence coding for antibody binding sites can be excised from the chromosome DNA and inserted into a cloning vector, which can be expressed in bacteria to produce recombinant proteins having the corresponding antibody binding sites.

In general, antibodies can be purified by known techniques such as chromatography, e.g., DEAE chromatography, ABx chromatography, and the like, filtration, and so forth.

An analyte analog is a modified analyte, which can compete with the analogous analyte for a receptor, the modification providing means to join an analyte analog to another molecule. The analyte analog will usually differ from the analyte by more than replacement of a hydrogen with a bond that links the analyte analog to a hub or label, but need not. The analyte analog can bind to the receptor in a manner similar to the analyte. The analog may be, for example, a label conjugate of the analyte, an antibody directed against the idiotype of an antibody to the analyte and the like.

As indicated above, analyte analogs include label conjugates, which may be prepared from certain of the haptens described above by incorporation of a desired label. The two components may be bound together, optionally through a linking group, to form a single structure. The binding can be either covalent attachment such as by a direct connection, e.g., a chemical bond, between the components or between the components and a linking group or non-covalent attachment involving specific binding between complementary specific binding pair (sbp) members that are attached to components. The procedures employed for the conjugation are well-known in the art.

Typically, for covalent attachment, one or more of the components contains a functional group suitable for attachment to one or more of the other components. The functional groups suitable for attaching the components may be carbonyl functionalities, both oxocarbonyl, e.g., aldehyde, and non-oxocarbonyl (including nitrogen and sulfur analogs) e.g., carboxy, amidine, amidate, thiocarboxy and thionocarboxy. Alternative functionalities of oxo include active halogen, diazo, mercapto, olefin, particularly activated olefin, amino, phosphoro and the like. Of particular interest are activated esters or alkylating agents. Details of techniques for attaching molecules to one another are well known in the art. See, for example, Matthews, et al., Anal. Biochem. (1985) 151:205-209; Engelhardt, et al., European Patent Application No. 0302175 and U.S. Patent No. 3,817,837.

As indicated above, the components, i.e., hapten and label, of the reagents may be attached together non-covalently. For example, a small organic molecule such as, for example, biotin including bis-biotin, fluorescein or the like may be incorporated into one of the components and the other component may be linked to a binding partner for the small organic molecule such as, for example, respectively, streptavidin, anti-fluorescein or the like. The binding of the binding partners results in the non-covalent attachment of the components to one another.

### Assays

The aforementioned reagents may be employed in all types of immunoassays to determine the presence and/or amount of entactogen analytes in a sample suspected of containing such analytes. The immunoassays may involve labeled or non-labeled reagents. Immunoassays involving non-labeled reagents usually comprise the formation of relatively large antigen-antibody complexes. Such assays include, for example, immunoprecipitin and agglutination methods and corresponding light scattering techniques such as, e.g., nephelometry and turbidimetry, for the detection of antigen-antibody complexes. Labeled immunoassays include enzyme immunoassays, fluorescence polarization immunoassays, radioimmunoassay, inhibition assay, luminescent oxygen channeling assay, and so forth.

One general group of immunoassays includes immunoassays of antigens or haptens using labeled analyte with a limited concentration of antibody. Another group of immunoassays involves the use of an excess of all of the principal reagents. Such assays include two-site sandwich assays, e.g., immunoradiometric assays, immunofluorometric assays, immunochemiluminometric assays, ELISA assays, and so forth. Another group of immunoassays includes precipitation, nephelometric and turbidimetric immunoassays, particle agglutination immunoassays, particle counting immunoassays, and the like. Another group of immunoassays are separation-free homogeneous assays in which the labeled reagents modulate the label signal upon antigen-antibody binding reactions. Another group of assays includes labeled antibody reagent limited competitive assays for hapten or antigen that avoid the use of problematic labeled antigens or haptens. In this type of assay, it is important that the solid phase immobilized analyte be present in a constant, limited amount. The partition of a label between the immobilized analyte and free analyte depends on the concentration of analyte in the sample.
The aforementioned haptens, label conjugates and antibodies may be employed to conduct an immunoassay for the entactogen analytes MDA, MDMA, HMMA and/or MDEA. The assays can be performed either without separation (homogeneous) or with separation (heterogeneous) of any of the assay components or products. Homogeneous immunoassays are exemplified by the EMIT® assay (Syva Company, San Jose, CA) disclosed in Rubenstein, et al., U.S. Patent No. 3,817,837, column 3, line 6 to column 6, line 64; immunofluorescence methods such as those disclosed in Ullman, et al., U.S. Patent No. 3,996,345, column 17, line 59, to column 23, line 25; enzyme channeling immunoassays ("ECIA") such as those disclosed in Maggio, et al., U.S. Patent No. 4,233,402, column 6, line 25 to column 9, line 63; the fluorescence polarization immunoassay ("FPIA") as disclosed, for example, in, among others, U.S. Patent No. 5,354,693; and so forth.

Other enzyme immunoassays are the enzyme modulate mediated immunoassay ("EMMIA") discussed by Ngo and Lenhoff, FEBS Lett. (1980) 116:285-288; the substrate labeled fluorescence immunoassay ("SLFIA") disclosed by Oellerich, J. Clin. Chem. Clin. Biochem. (1984) 22:895-904; the combined enzyme donor immunoassays ("CEDIA") disclosed by Khanna, et al., Clin. Chem. Acta (1989) 185:231-240; homogeneous particle labeled immunoassays such as particle enhanced turbidimetric inhibition immunoassays ("PETINIA"), particle enhanced turbidimetric immunoassay ("PETIA"), etc.; and the like.

Exemplary of heterogeneous assays are the enzyme linked immunosorbant assay ("ELISA") discussed in Maggio, E.T. *supra*; the radioimmunoassay, disclosed in Yalow, et al., J. Clin. Invest. 39:1157 (1960) and so forth.

Other assays include the sol particle immunoassay ("SPIA"), the disperse dye immunoassay ("DIA"); the metalloimmunoassay ("MIA"); the enzyme membrane immunoassays ("EMIA"); luminoimmunoassays ("LIA"); and so forth. Other types of assays include immunosensor assays involving the monitoring of the changes in the optical, acoustic and electrical properties of an antibody-immobilized surface upon the binding of an antigen or hapten. Such assays include, for example, optical immunosensor assays, acoustic immunosensor assays, semiconductor immunosensor assays, electrochemical transducer immunosensor assays, potentiometric immunosensor assays, amperometric electrode assays, and the like.

The above reagents may also be employed in multi-analyte immunoassays where one or more entactogen analytes may be the subject of detection along with one or more other analytes such as other drugs of abuse and the like. Such multi-analyte systems are described in U.S. Patent No. 5,135,836.

The homogeneous or heterogeneous assays, particularly enzyme immunoassays and fluorescence polarization immunoassays, are normally carried out in an aqueous buffered medium at a moderate pH, generally that which provides optimum assay sensitivity. The aqueous medium may be solely water or may include from 0 to about 40 volume percent of a cosolvent. The pH for the medium will usually be in the range of about 4 to about 11, more usually in the range of about 5 to about 10, and preferably in the range of about 6.5 to about 9.5. The pH will usually be a compromise between optimum binding of the binding members of any specific binding pairs, the pH optimum for other reagents of the assay such as members of the signal producing system, and so forth.

Various buffers may be used to achieve the desired pH and maintain the pH during the determination. Illustrative buffers include borate, phosphate, carbonate, tris, barbital and the like. The particular buffer employed is not critical to this invention, but in an individual assay one or another buffer may be preferred. Various ancillary materials may be employed in the method in accordance with the present invention. For example, in addition to buffers the medium may comprise stabilizers for the medium and for the reagents employed. Frequently, in addition to these additives, proteins may be included, such as albumins; organic solvents such as formamide; quaternary ammonium salts; polyanions such as dextran sulfate; surfactants, particularly non-ionic surfactants; binding enhancers, e.g., polyalkylene glycols; or the like.

One or more incubation periods may be applied to the medium at one or more intervals including any intervals between additions of various reagents mentioned above. The medium is usually incubated at a temperature and for a time sufficient for binding of various components of the reagents to occur. Moderate temperatures are normally employed for carrying out the method and usually constant temperature, preferably, room temperature, during the period of the measurement. Incubation temperatures normally range from about 5° to about 99°C, usually from about 15 °C to about 70 °C, more usually 20 °C to about 45 °C. The time period for the incubation is about 0.2 seconds to about 6 hours, usually, from about 2 seconds to about 1 hour, more usually, about 1 to about 5 minutes. The time period depends on the temperature of the medium and the rate of binding of the various reagents, which is determined by the association rate constant, the concentration, the binding constant and dissociation rate constant. Temperatures during measurements will generally range from about 10 to about 50 °C, more usually from about 15 to about 40 °C.

The concentration of entactogen analyte that may be assayed generally varies from about 10⁻⁵ to about 10⁻¹⁷ M, more usually from about 10⁻⁶ to about 10⁻¹⁴ M. Considerations, such as whether the assay is qualitative, semi-quantitative or quantitative (relative to the amount of analyte present in the sample), the particular detection technique and the concentration of the analyte will normally determine the concentrations of the various reagents.

The concentration of analytes to be detected will generally vary from about 10⁻⁵ to about 10⁻¹⁷ M, more usually from about 10⁻⁶ to about 10⁻¹⁴ M. In general, a predetermined cut-off level is established for each analyte suspected of being in a sample. The particular predetermined cut-off level generally is determined on an analyte by analyte basis. Those skilled in the art are well aware of the factors relating to the selection of predetermined cut-off levels. For example, for many drugs of abuse, the cut-off levels are determined by SAMSA, an agency of the Department of Health and Human Services. The nature of the signal producing system may be a consideration in determining the predetermined cut-off levels of some analytes. Another consideration is that the expected variation in concentration of the analytes that is of significance should provide an accurately measurable signal difference.

The concentrations of the various reagents in the assay medium will generally be determined by the concentration range of interest of the entactogen analyte. However, the final concentration of each of the reagents will normally be determined empirically to optimize the sensitivity of the assay over the range. That is, a variation in concentration of entactogen analyte that is of significance should provide an accurately measurable signal difference. Considerations such as the nature of the signal producing system and the nature of, and predetermined cut-off levels for, the entactogen analytes normally determine the concentrations of the various reagents.

While the order of addition may be varied widely, there will be certain preferences depending on the nature of the assay. The simplest order of addition is to add all the materials simultaneously and determine the effect that the assay medium has on the signal as in a homogeneous assay. Alternatively, the reagents can be combined sequentially. Optionally, an incubation step may be involved subsequent to each addition, generally ranging from about 30 seconds to about 6 hours, more usually from about 1 minute to about 1 hour.

The following examples further describe the specific embodiments of the invention by way of illustration and not limitation and are intended to describe and not to limit the scope of the invention.

In a homogeneous assay after all of the reagents have been combined, the signal is determined and related to the amount of entactogen analyte in the sample. For example, in an EMIT assay for MDA, a sample suspected of containing MDA is combined in an aqueous medium either simultaneously or sequentially with an MDA-enzyme conjugate and antibody capable of recognizing MDA and the conjugate, both of which are prepared in accordance with the present invention. Generally, a substrate for the enzyme is added, which results in the formation of a chromogenic or fluorogenic product upon enzyme catalyzed reaction. Preferred enzymes are glucose-6-phosphate dehydrogenase and alkaline phosphatase but other enzymes may be employed. The MDA and the MDA-enzyme conjugate compete for binding sites on the antibody. The enzyme activity in the medium is then determined, usually by spectrophotometric means, and is compared to the enzyme activity determined when calibrators or reference samples are tested in which a known amount of MDA is present. Typically, the calibrators are tested in a manner similar to the testing of the sample suspected of containing MDA. The calibrators will typically contain differing, but known, concentrations of the MDA analyte to be determined. Preferably, the concentration ranges present in the calibrators will span the range of suspected MDA concentrations in the unknown samples.

Heterogeneous assays usually involve one or more separation steps and can be competitive or non-competitive. A variety of competitive and non-competitive assay formats are disclosed in Davalian, et al., U.S. Patent No. 5,089,390, column 14, line 25 to column 15, line 9. In a typical competitive assay a support having an antibody for an entactogen analyte such as, for example, an antibody for MDA, bound thereto is contacted with a medium containing the sample and an MDA-label conjugate where MDA is conjugated to a detectable label such as an enzyme. After separating the support and the medium, the label activity of the support or the medium is determined by conventional techniques and related to the amount of MDA in the sample.

The support may be comprised of an organic or inorganic, solid or fluid, water insoluble material, which may be transparent or partially transparent. The support can have any of a number of shapes, such as particle, including bead, film, membrane, tube, well, strip, rod, plate and the like. Depending on the type of assay, the support may or may not be suspendable in the medium in which it is employed. Examples of suspendable supports are polymeric materials such as latex, lipid bilayers or liposomes, oil droplets, cells and hydrogels. Other support compositions include polymers, such as nitrocellulose, cellulose acetate, poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), etc.; either used by themselves or in conjunction with other materials.

Binding of components to the surface of a support may be direct or indirect, covalent or non-covalent and can be accomplished by well-known techniques, commonly available in the literature, as discussed above. See, for example, "Immobilized Enzymes," Ichiro Chibata, Halsted Press, New York (1978) and Cautrecasas, J. Biol. Chem., 245:3059 (1970). The surface of the support is usually polyfunctional or be capable of being polyfunctionalized or be capable of binding to an sbp member, or the like, through covalent or specific or non-specific non-covalent interactions. Such binding is indirect where non-covalent interactions are used and is direct where covalent interactions are employed. A wide variety of functional groups are available or can be incorporated. Functional groups include carboxylic acids, aldehydes, amino groups, cyano groups, ethylene groups, hydroxyl groups, mercapto groups and the like. The manner of linking a wide variety of compounds to surfaces is well known and is amply illustrated in the literature (see above).

Binding of the antibody for MDA and MDA results in the formation of an immune complex that can be detected directly or indirectly in numerous ways that are well known in the art. The immune complexes are detected directly, for example, when the antibodies employed are conjugated to a label. The immune complex is detected indirectly by examining for the effect of immune complex formation in an assay medium on a signal producing system or by employing a labeled receptor that specifically binds to an antibody produced by employing one of the hapten immunogen conjugates of the invention.

Activation of the signal producing system depends on the nature of the signal producing system members. For those members of a signal producing system that are activated with light, the member is irradiated with light. For members of signal producing systems that are on the surface of a particle, addition of a base may result in activation. Other activation methods will be suggested to those skilled in the art in view of the disclosures herein. For some signal producing systems no agent for activation is necessary such as those systems that involve a label that is a radioactive label, an enzyme, and so forth. For enzyme systems addition of a substrate and/or a cofactor may be necessary.

In certain embodiments first and second labels may be employed and comprise a label pair. These label pairs may be, for example, a singlet oxygen generator or sensitizer and chemiluminescent reactant pair, an enzyme pair wherein a product of the first enzyme serves as a substrate for the second enzyme and a luminescent energy donor and acceptor pair, e.g., an energy donor or acceptor and a fluorescent compound. The signal will usually be initiated by and/or detected as electromagnetic radiation and will preferably be luminescence such as chemiluminescence, fluorescence, electroluminescence or phosphorescence.

The examination for presence and amount of the signal also includes the detection of the signal, which is generally merely a step in which the signal is read. The signal is normally read using an instrument, the nature of which depends on the nature of the signal. The instrument may be a spectrophotometer, fluorometer, absorption spectrometer, luminometer, chemiluminometer, actinometer, photographic instrument, and the like. The presence and amount of signal detected is related to the presence and amount of the entactogen analyte present in a sample above the predetermined cut-off level. Temperatures during measurements generally range from about 10° to about 70°C, more usually from about 20° to about 45°C, more usually about 20° to about 25°C. In one approach standard curves are formed using known concentrations of the analytes to be screened. Calibrators and other controls may also be used.

The description below of certain exemplary embodiments of methods uses the language "and/or," which means that the method may or may not involve each item mentioned. This language is used for the sake of brevity. In general, a method will involve at least one antibody for an analyte, e.g., methylenedioxyamphetamine, and at least one enzyme conjugate that corresponds to that analyte, e.g., an enzyme conjugate of a methylenedioxyamphetamine.

One embodiment is a method for determining amphetamine and/or methamphetamine and/or methylenedioxyethamphetamine in a sample suspected of containing methylenedioxyamphetamine and/or methylenedioxymethamphetamine and/or methylenedioxyethamphetamine, said method comprising:
(a) providing in combination in a medium:
   (i) said sample,
   (ii) an antibody for methylenedioxyamphetamine, and/or
   (iii) an antibody for methylenedioxymethamphetamine, and/or
   (iv) an antibody for methylenedioxyethamphetamine, and
   (v) a compound of the formula: 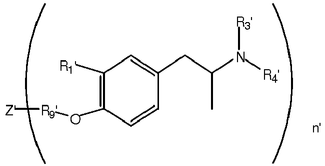 wherein:
      R¹' is H, or methyl or ethyl, preferably, H,
      R³' is H,
      R⁴' is H, or methyl or ethyl,
      R⁹' is -(CH₂)ₙC(O)R⁶' or -(CH₂)ₙR⁶',
      R⁶' is Z', which is an enzyme, such as, e.g., G6PDH,
      n' is an integer between 1 and the molecular weight of said enzyme divided by about 500; and
(b) examining said medium for the presence of a complex comprising said methylenedioxyamphetamine and said antibody for methylenedioxyamphetamine and/or a complex of said methylenedioxymethamphetamine and said antibody for methylenedioxymethamphetamine and/or a complex of said methylenedioxyethamphetamine and said antibody for methylenedioxyethamphetamine, the presence thereof indicating the presence of said methylenedioxyamphetamine and/or methylenedioxymethamphetamine and/or methylenedioxyethamphetamine in said sample.

The examining may comprise measuring signal from the enzyme, the amount thereof being related to the presence of the methylenedioxyamphetamine and/or methylenedioxymethamphetamine and/or methylenedioxyethamphetamine in the sample. The method may be a homogeneous method and the medium is examined for the amount of the signal. The method may be a heterogeneous method and the complex, if present, is separated from the medium and the medium or the complex is examined for the amount of the signal. The enzyme may be glucose-6-phosphate dehydrogenase.

One embodiment is a method for determining methylenedioxyamphetamine and/or methylenedioxymethamphetamine and/or methylenedioxyethamphetamine in a sample suspected of containing methylenedioxyamphetamine and/or methylenedioxy-methamphetamine and/or methylenedioxyethamphetamine, said method comprising:
(a) providing in combination in a medium:
   (i) said sample,
   (ii) a conjugate of an enzyme and a methylenedioxyamphetamine analog and/or a conjugate of an enzyme and a methylenedioxymethamphetamine analog and/or a conjugate of an enzyme and a methylenedioxyethamphetamine analog,
   (iii) an antibody for methylenedioxyamphetamine, said antibody being raised against a compound of the formula: 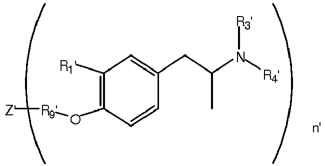 wherein:
      R¹' is H, or methyl or ethyl, preferably, H,
      R³' is H,
      R⁴' is H,
      R⁹' is -(CH₂)ₙC(O)R⁶' or -(CH₂)ₙR⁶',
      R⁶' is Z', which is an immunogenic protein, such as, e.g., -NH-protein, or a non-poly(amino acid) immunogenic carrier,
      n' is an integer between 1 and the molecular weight of said immunogenic protein or said immunogenic carrier divided by about 500; and/or
   (iv) an antibody for methylenedioxymethamphetamine, said antibody being raised against a compound of the formula: 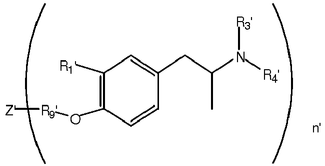 wherein:
      R¹' is H, or methyl or ethyl, preferably, H,
      R³' is H,
      R⁴' is methyl,
      R⁹' is -(CH₂)ₙC(O)R⁶' or -(CH₂)ₙR⁶',
      R⁶' is Z', which is an immunogenic protein, such as, e.g., -NH-protein, or a non-poly(amino acid) immunogenic carrier,
      n' is an integer between 1 and the molecular weight of said immunogenic protein or said immunogenic carrier divided by about 500; and/or
   (v) an antibody for methylenedioxyethamphetamine, said antibody being raised against a compound of the formula: 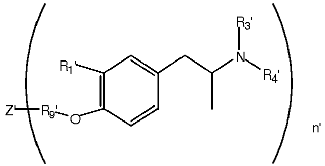 wherein:
      R¹' is H, or methyl or ethyl, preferably, H,
      R³' is H,
      R⁴' is ethyl,
      R⁹' is -(CH₂)ₙC(O)R⁶' or -(CH₂)ₙR⁶',
      R⁶' is Z', which is an immunogenic protein, such as, e.g., -NH-protein, or a non-poly(amino acid) immunogenic carrier,
      n' is an integer between 1 and the molecular weight of said immunogenic protein or said immunogenic carrier divided by about 500; and
(b) examining said medium for the presence of a complex comprising said methylenedioxyamphetamine and said antibody for methylenedioxyamphetamine and/or a complex of said methylenedioxymethamphetamine and said antibody for methylenedioxymethamphetamine and/or a complex of said methylenedioxyethamphetamine and said antibody for methylenedioxyethamphetamine, the presence thereof indicating the presence of said methylenedioxyamphetamine and/or methylenedioxymethamphetamine and/or methylenedioxyethamphetamine in said sample.

The examining may comprise measuring signal from the enzyme, the amount thereof being related to the presence of the methylenedioxyamphetamine and/or methylenedioxymethamphetamine and/or methylenedioxyethamphetamine in the sample. The method may be a homogeneous method and the medium is examined for the amount of the signal. The method may be a heterogeneous method and the complex, if present, is separated from the medium and the medium or the complex is examined for the amount of the signal. The enzyme may be glucose-6-phosphate dehydrogenase.

### Kits

Another aspect of the present invention relates to kits useful for conveniently performing an assay for the determination of an entactogen analyte such as, for example, 3,4-methylenedioxyamphetamine (MDA), 3,4-methylenedioxy-methamphetamine (MDMA), 3,4-methylenedioxyethyl-amphetamine (MDEA) and 4-hydroxy-3-methoxy-methamphetamine (HMMA). The kit comprises (a) an antibody raised against a conjugate of a protein and a compound of Formula I and (b) ancillary reagents for determining the compound. The kit may further comprise a label conjugate of the compound of Formula I above.

To enhance the versatility of the subject invention, the kit reagents can be provided in packaged combination, in the same or separate containers, in liquid or lyophilized form so that the ratio of the reagents provides for substantial optimization of the method and assay. The reagents may each be in separate containers or various reagents can be combined in one or more containers depending on the cross-reactivity and stability of the reagents.

The kit can further include other separately packaged reagents for conducting an assay such as additional sbp members, ancillary reagents such as an ancillary enzyme substrate, and so forth. The relative amounts of the various reagents in the kits can be varied widely to provide for concentrations of the reagents that substantially optimize the reactions that need to occur during the present method and to further substantially optimize the sensitivity of the assay. Under appropriate circumstances one or more of the reagents in the kit can be provided as a dry powder, usually lyophilized, including excipients, which on dissolution will provide for a reagent solution having the appropriate concentrations for performing a method or assay in accordance with the present invention. The kit can further include a written description of a method in accordance with the present invention as described above.

The description below of certain exemplary embodiments of kits uses the language "and/or," which means that the kit may or may not contain each item mentioned. This language is used for the sake of brevity. In general, a kit will include at least one antibody for an analyte, e.g., methylenedioxyamphetamine, and at least one enzyme conjugate that corresponds to that analyte, e.g., an enzyme conjugate of a methylenedioxyamphetamine.

A particular embodiment is a kit comprising in packaged combination:
(i) an antibody for methylenedioxyamphetamine,
(ii) an antibody for methylenedioxymethamphetamine, and/or
(iii) an antibody for methylenedioxyethamphetamine, and
(iv) a compound of the formula: 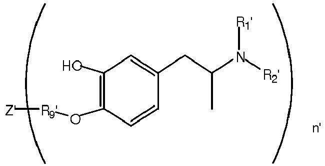 wherein:
   R¹' is H,
   R²' is H, or methyl or ethyl,
   R⁹' is -(CH₂)ₙC(O)R⁵' or -(CH₂)ₙR⁵',
   R⁵' is Z', which is an immunogenic protein or a non-poly(amino acid) immunogenic carrier,
   n' is an integer between 1 and the molecular weight of said immunogenic protein or said immunogenic carrier divided by about 500.

Another embodiment of a kit comprises in packaged combination:
(i) a conjugate of an enzyme and a methylenedioxyamphetamine analog
   and/or a conjugate of an enzyme and a methylenedioxymethamphetamine analog, and/or a conjugate of an enzyme and a methylenedioxyethamphetamine analog, and
(ii) an antibody for methylenedioxyamphetamine, said antibody being raised against a compound of the formula: 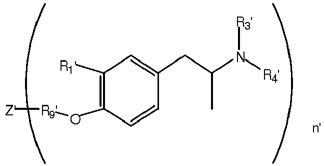 wherein:
   R¹' is H, methyl or ethyl, preferably, H,
   R³' is H,
   R⁴' is H,
   R⁹' is -(CH₂)ₙC(O)R⁶' or -(CH₂)ₙR⁶',
   R⁶' is Z', which is an immunogenic protein, such as, e.g., -NH-protein, or a non-poly(amino acid) immunogenic carrier,
   n' is an integer between 1 and the molecular weight of said immunogenic protein or said immunogenic carrier divided by about 500; and/or
iii) an antibody for methylenedioxymethamphetamine, said antibody being raised against a compound of the formula: 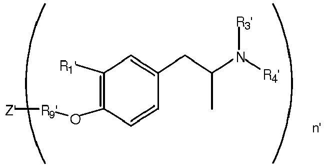 wherein:
   R¹' is H, methyl or ethyl, preferably, H,
   R³' is H,
   R⁴' is methyl,
   R⁹' is -(CH₂)ₙC(O)R⁶' or -(CH₂)ₙR⁶',
   R⁶' is Z', which is an immunogenic protein, such as, e.g., -NH-protein, or a non-poly(amino acid) immunogenic carrier,
   n' is an integer between 1 and the molecular weight of said immunogenic protein or said immunogenic carrier divided by about 500, and/or
(iv) an antibody for methylenedioxyethamphetamine, said antibody being raised against a compound of the formula: 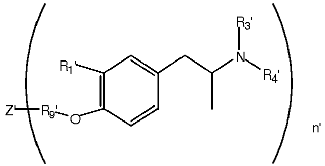 wherein:
   R¹' is H, methyl or ethyl, preferably, H,
   R³' is H,
   R⁴' is ethyl,
   R⁹' is -(CH₂)ₙC(O)R⁶' or -(CH₂)ₙR⁶',
   R⁶' is Z', which is an immunogenic protein, such as, e.g., -NH-protein, or a non-poly(amino acid) immunogenic carrier,
   n' is an integer between 1 and the molecular weight of said immunogenic protein or said immunogenic carrier divided by about 500.

### EXAMPLES

The invention is demonstrated further by the following illustrative examples. Parts and percentages recited herein are by weight unless otherwise specified. Temperatures are in degrees centigrade (°C).

Analytical thin layer chromatography (TLC) was the usual analysis method and performed on Analtech Uniplate Silica Gel GF (0.25 mm) glass-backed plates using the specified solvent. The spots on TLC were visualized by ultraviolet light (short and /or long wave) and /or iodine vapors. Flash chromatography was carried out on Whatman silica gel 60 Å (230-400 mesh). All chemicals were obtained from Sigma Chemical Company (St. Louis, MO), Aldrich Chemical Company (St. Louis, MO), Fluka (Milwaukee, WI) and used as received. ¹H-NMR and ¹³C-NMR spectra routinely recorded on a Bruker Ultrashiel™-400 (400 MHz) spectrometer (Bruker, Billerica MA). Chemical shift were reported in parts per million (ppm, δ) and related to tetramethylsilane or with deuterated solvent as internal reference. NMR abbreviations used are s (singlet), d (doublet), and m (multiplet). Mass spectra were obtained at the Mass Spectrometry Laboratory, University of California at Berkeley, Berkeley, California.

Melting points were determined on a Hoover capillary apparatus and are uncorrected. Infrared spectra were recorded on a Perkin-Elmer 297IR spectrometer. UV-visible absorption spectra were done on a HP 8452A diode array spectrophotometer. Fluorescence measurements were done on a Spex fluorolog spectrophotometer or a Perkin Elmer 650-40 spectrophotometer.

The following abbreviations have the meanings set forth below:
g - grams
mg - milligrams
mL - milliliters
µL - microliters
mmol - millimoles
DMF - dimethyl formamide
THF - tetrahydrofuran
NMR - nuclear magnetic resonance spectroscopy
MHz - megahertz
EDAC - 1-ethyl-3-(3-dimethylaminopropyl)carbodiimide hydrochloride (Sigma Chemical Company)
MeOH - methanol
FAB-MS - fast atom bombardment - mass spectrometry
DI water - deionized water
BCA Protein Concentration Assay - Pierce Chemical Company
TNBS - 2,4,6-trinitrobenzene sulfonic acid
KLH - keyhole limpet hemocyanin
NHS - N-hydroxysuccinimic ester
THF - tetrahydrofuran
tBoc₂O - di-tert-butyldicarbonate
TFA - trifluoroacetic acid

### Preparation of Antibodies

The following methods may be employed to prepare polyclonal antibodies: Antiserum containing antibodies is obtained by well-established techniques involving immunization of an animal, such as a rabbit, guinea pig, or goat, with an appropriate immunogen and obtaining antisera from the blood of the immunized animal after an appropriate waiting period. State-of-the-art reviews are provided by Parker, Radioimmunoassay of Biologically Active Compounds, Prentice-Hall (Englewood Cliffs, N.J., U.S., 1976), Butler, J. Immunol. Meth. 7: 1-24 (1975); Broughton and Strong, Clin. Chem. 22: 726-732 (1976); and Playfair, et al., Br. Med. Bull. 30: 24-31 (1974).

The following procedure may be employed to prepare monoclonal antibodies: Monoclonal antibodies are produced according to the standard techniques of Köhler and Milstein, Nature 265:495-497, 1975. Reviews of monoclonal antibody techniques are found in Lymphocyte Hybridomas, ed. Melchers, et al. Springer-Verlag (New York 1978), Nature 266: 495 (1977), Science 208: 692 (1980), and Methods of Enzymology 73 (Part B): 3-46 (1981). Samples of an appropriate immunogen preparation are injected into an animal such as a mouse and, after a sufficient time, the animal is sacrificed and spleen cells obtained. Alternatively, the spleen cells of a non-immunized animal can be sensitized to the immunogen *in vitro.* The spleen cell chromosomes encoding the base sequences for the desired immunoglobulins can be compressed by fusing the spleen cells, generally in the presence of a non-ionic detergent, for example, polyethylene glycol, with a myeloma cell line. The resulting cells, which include fused hybridomas, are allowed to grow in a selective medium, such as HAT-medium, and the surviving immortalized cells are grown in such medium using limiting dilution conditions. The cells are grown in a suitable container, e.g., microtiter wells, and the supernatant is screened for monoclonal antibodies having the desired specificity.

Various techniques exist for enhancing yields of monoclonal antibodies, such as injection of the hybridoma cells into the peritoneal cavity of a mammalian host, which accepts the cells, and harvesting the ascites fluid. Where an insufficient amount of the monoclonal antibody collects in the ascites fluid, the antibody is harvested from the blood of the host. Alternatively, the cell producing the desired antibody can be grown in a hollow fiber cell culture device or a spinner flask device, both of which are well known in the art. Various conventional ways exist for isolation and purification of the monoclonal antibodies from other proteins and other contaminants (see Köhler and Milstein, *supra*).

In general, antibodies can be purified by known techniques such as chromatography, e.g., DEAE chromatography, ABx chromatography, and the like, filtration, and so forth.

### Preparation of compound (24)

To a solution of methylamine, hydrochloride (4.1 g, 60.72 mmol) in MeOH (50 mL) was added NA_{A}CO (6.2 g, 58.5 mmol). The reaction was stirred at room temperature for 30 minutes. The reaction mixture was filtered through into a 100 mL of roundbottom flask containing 4-hydroxy-3-methyl phenyl acetone (**22**) (1.8 g, 10 mmol). The reaction mixture was refluxed for 2 hours and allowed to cool to room temperature. Sodium cyanoborohydride (628 mg, 10 mmol) was added to the mixture. The reaction mixture was refluxed for 5 hours and during this time the pH of the solution was maintained at neutrality by addition of 4M HCI in dioxane. The organic solvent was evaporated to dryness by rotary evaporation and the residue was dissolved in 20 mL of water. The solution was acidified with 6 N HCI to pH = 2-3, extracted with ethyl acetate, then basified to pH = 9-10 with 6 N NaOH, saturated with NaCl. The mixture was extracted with ethyl acetate, the combined organic extracts were dried over anhydrous MgSO₄ and filtered. The ethyl acetate was removed by evaporation to give an oil. The oily residue was purified by flash column chromatography (silica gel) using MeOH/CH₂Cl₂ (1/4) as an eluent to give the desired product (**24**) (532 mg, 27% yield); ¹H-NMR (CDCl₃, 400 MHz) δ: 6.82 (d, *J* = 7.9 Hz, 1H), 6.67 (s, 1H), 6.65 (d, *J* = 7.4 Hz, 1H), 4.82 (s, OH), 3.85 (s, 3H), 3.46 (s, 3H), 2.75 (m, 1H), 2.587(m, 2H), 1.06 (d, *J* = 6.2 Hz, 3H);

### Preparation of compound (25)

To a solution of **24** (513 mg, 2.627 mmol) in THF (30 mL) and H₂O (20 mL) was added tBoc₂O (1.21 g, 5.25 mmol) and K₂CO₃ (1.09 g, 7.89 mmol). The reaction was stirred at room temperature for 4 hours. Water (20 mL) was added and most of THF was removed by rotary evaporation. The aqueous phase was extracted with ethyl acetate (3 x 40 mL). The combined organic solvent was washed with water (30 mL) and dried over MgSO₄. The solvent was filtered and concentrated to dryness. The residue was purified by flash column chromatography using ethyl acetate/hexane (1/4) as an eluent to give the desired product (**25**) (632 mg, 82%); FAB-MS: MH⁺ (296); ¹H-NMR (CDCl₃, 400 MHz) δ: 6.80-6.52 (m, 3H), 5.96 (m, 1H, OH), 4.48, 4.19 (s, 1H), 3.78 (s, 3H), 2.68-2.50 (m, 5H), 1.48-1.46(m, 9H), 1.07-1.04 (m, 3H).

### Preparation of compound (26)

To a solution of **25** (100 mg, 0.3385 mmol) in DMF (10 mL) was added NaH (77 mg, 3.05 mmol). The reaction mixture was stirred at room temperature for 30 minutes. Methyl bromoacetate (73 mg, 0.474 mmol) was added to the mixture. The mixture was stirred at room temperature for 65 hours. DMF was removed by rotary evaporation and water (10 mL) was added. The aqueous phase was extracted with ethyl acetate (3 x 30 mL). The combined organic phases were washed with water (15 mL) and dried over MgSO₄. The organic phase were filtered and concentrated to dryness. The residue was purified by flash column chromatography using ethyl acetate/hexane (3/7) as an eluent to give the desired product (**26**) (38 mg, 31% yield); ¹H-NMR (CDCl₃, 400 MHz) δ: 6.73 (m, 3H), 4.65 (s, 2H), 4.51, 4.24 (s, 1H), 3.86 (s, 3H), 3.77 (s, 3H), 2.79-2.55 (m, 5H), 1.38-1.32(m, 9H), 1.13 (bs, 3H).

### Preparation of compound (27)

To a solution of **26** (19 mg, 0.0517 mmol) in CH₂Cl₂ (2 mL) was added TFA (0.3 mL). The reaction mixture was stirred at room temperature for 120 minutes. TLC analysis of the reaction showed that starting material (**26**) disappeared and a new, polar spot displayed (silica gel, ethyl acetate/hexane = 2/3). Most of CH₂Cl₂ and TFA were removed by rotary evaporation under reduced pressure. The residue was put in high vacuum to remove trace amount of TFA. This gave the desired product (27) (19 mg, 96% yield); ¹H-NMR (CDCl₃, 400 MHz) δ: 7.54 (bs, NH), 6.72 (m, 3H), 4.66 (s, 2H), 3.83 (s, 3H), 3.79 (s, 3H), 3.40 (m, 1H), 3.03 (1H), 2.79 (m, 1H), 2.72 (bs, 3H), 1.32 (d, *J* = 6.4 Hz, 3H).

### Preparation of compound (28)

To a solution of **27** (18 mg, 0.0472 mmol) in MeOH (5 mL) and water (1 mL) was added K₂CO₃ (33 mg, 0.239 mmol). The reaction mixture was stirred at room temperature for 180 minutes. 1 N HCl was added to adjust the pH values to 2-3. Most of MeOH, HCl and water were removed by rotary evaporation under vacuum. The residue was purified by flash column chromatography (silica gel) using MeOH/CH₂Cl₂/AcOH (2/8/0.1) to give the desired product (**28**) (12 mg, 88% yield); ¹H-NMR (D₂O, 400 MHz) δ: 6.77 (m, 3H), 4.58 (s, 2H), 3.72 (s, 3H), 3.38 (m, 1H), 2.87 (m, 1H), 2.71 (m, 1H), 2.57 (s, 3H), 1.13 (d, *J* = 6.5 Hz, 3H).

### Preparation of HMMA Immunogen (30)

To a solution of **28** (10 mg, 0.0345 mmol) in DMF (0.6 mL) was added EDAC (20 mg, 0.1043 mmol) and NHS (19 mg, 0.165 mmol). The reaction was stirred at room temperature under argon for 2.5 hours. The activated hapten (**29**) was added drop wise under argon to 6 mL of sodium phosphate solution (0.1 M, pH = 8.0) of KLH (20 mg) at 0 °C under argon. The pH value changed during the addition and 0.1 N of NaOH aqueous solution was used to maintain the pH to 8.0. After completed the addition, the conjugate was allowed to stir at 4 °C for 16 hours. The conjugate was dialyzed against Dulbecco's phosphate buffered saline (pH = 7.0, 3 Liters) prepared from Dulbecco's phosphate buffered saline (Sigma buffer, 400 mL) diluting with DE water (2600 mL) at 4 °C for 4 hours. The dialyzing procedure was repeated with fresh buffer solution for 16, 24 and 40 hours. Finally, the conjugate was dialyzed with sodium phosphate buffer solution (10 mM, pH = 7.0) two times (3 hours and 4 hours). The concentration of protein was measured by using BCA Protein Concentration Assay and the TNBS method was used for hapten number determination. The immunogen (**30**) has a concentration of 2.12 mg/mL with the hapten number of 1436, and used for the immunization of rats for antibody production.

### Preparation of compound (31)

To a solution of **25** (86 mg, 0.291 mmol) in toluene (15 mL) was added K₂CO₃ (200 mg) and dibromoethane (2 mL). The reaction was refluxed for 48 hours and stirred at room temperature for 66 hours. Water (10 mL) was added and toluene was separated. The aqueous phase was extracted with ethyl acetate (3 x 30 mL). The combined organic solvent was washed with water (20 mL) and dried over MgSO₄. The solvent was filtered and concentrated to dryness. The residue was purified by flash column chromatography using ethyl acetate/hexane (1/4) as an eluent to give the desired product (**31**) (62 mg, 53%); ¹H-NMR (CDCl₃, 400 MHz) δ: 6.78 (m, 3H), 4.26 (t, *J* = 6.6 Hz, 2H), 3.82 (s, 3H), 3.60 (t, *J* = 6.7 Hz, 2H), 2.67 (m, 5H), 1.36 (m, 9H), 1.11 (bs, 3H).

### Preparation of compound (32)

To a solution of **31** (61 mg, 0.1516 mmol) in 95% ethanol (10 mL) was added potassium thioacetate (100 mg, 0.8756 mmol). The reaction was stirred at 55 °C under argon for 3 hours. Most of ethanol was removed by rotary evaporation. The residue was re-dissolved in 5 mL of CH₂Cl₂ and the precipitate was filtered off and washed with CH₂Cl₂ (2 x 10 mL). The combined organic solvent was concentrated to dryness. The residue was purified by flash column chromatography using ethyl acetate/hexane (3/7) as an eluent to give the desired product (**32**) (50 mg, 83%); ¹H-NMR (CDCl₃, 400 MHz) δ: 6.82 (m, 1H), 6.64 (m, 2H), 4.08 (t, *J* = 6.7 Hz, 2H), 3.82 (s, 3H), 3.25 (t, *J* = 6.7 Hz, 2H), 2.65 (m, 5H), 2.33 (s, 3H), 1.33 (m, 9H), 1.10 (bs, 3H).

### Preparation of compound (33)

To a solution of **32** (48 mg, 0.1207 mmol) in CH₂Cl₂ (3 mL) was added TFA (0.4 mL). The reaction mixture was stirred at room temperature for 1.5 hours. TLC analysis of the mixture showed that starting material, **22** disappeared and a new spot displayed at baseline (silica gel, ethyl acetate/hexane = 1/1). Most of CH₂Cl₂ and TFA were removed by rotary evaporation. The residue was put in high vacuum to remove traces of TFA. This gave the desired product, (**33**) (49 mg, 98%); ¹H-NMR (CDCl₃, 400 MHz) δ: 6.86 (m, 1H), 6.68 (m, 2H), 4.09 (t, *J* = 6.7 Hz, 2H), 3.82 (s, 3H), 3.37 (m, 1H), 3.26 (t, *J* = 6.7 Hz, 2H), 3.07 (m, 1H), 2.70 (m, 4H), 2.34 (s, 3H), 1.28 (d, *J* = 6.4 Hz, 3H); ¹³C-NMR (CDCl₃, 100 MHz) δ: 196.3, 150.3, 147.6, 128.8, 121.8, 117.5, 114.5, 113.3, 68.0, 57.7, 56.4, 39.6, 30.9, 28.6, 15.8.

### Preparation of bromoacetyl-KLH (35)

To a solution of bromoacetic acid NHS ester (8.6 mg, 0.0364 mmol) in DMF (0.3 mL) was added to a solution of KLH (40 mg) in NaH₂PO₄-Na₂HPO₄ buffer (pH = 8.00, 0.1M, 4 mL) at 4 °C. The reaction mixture was stirred in the cold-room (4 °C) for 16 hours. The mixture was chromatographed on a packed Sephadex G-50, eluting with NaH₂PO₄-Na₂HPO₄ buffer (pH = 8.00, 0.1M) with a flow rate of 20 mL/hour. The eluted fractions (4 mL in volume for each fraction) from the column were monitored by UV at 280 nm. Fractions #10-13 were pooled to give 12 mL of bromoacetyl-KLH (**35**). The concentration of protein was measured by UV method and the TNBS method was used for hapten number determination. The conjugate has a concentration of 3.28 mg/mL with the hapten number of 925.

### Preparation of HMMA immunogen (36)

To a solution of **33** (25 mg, 0.06 mmol) in MeOH (0.5 mL) and water (0.1 mL) was added K₂CO₃ (20 mg). The reaction mixture was stirred at room temperature under argon for 1.5 hours. TLC analysis of the mixture showed that a new spot displayed to be product (**34**). ¹H-NMR (CDCl₃, 400 MHz) δ: 6.84 (m, 1H), 6.68 (m, 2H), 4.26 (m, 2H), 3.84 (s, 3H), 3.11 (m, 2H), 3.07 (m, 1H), 2.73 (m, 1H), 2.58 (m, 2H), 2.38 (s, 3H), 1.05 (d, *J* = 6.2 Hz, 3H).

To a solution of well-prepared bromoacetyl-KLH (**35**) (8 mL, 3.28 mg/mL, pH = 8.00) was added the above reaction mixture slowly at 4 °C under argon. The reaction was stirred at 4 °C for 16 hours. The reaction mixture was chromatographed on a Sephadex G-50 column, which was equilibrated with NaH₂PO₄-Na₂HPO₄ buffer (pH = 7.20, 0.1 M, 200 mL). The column was eluted with NaH₂PO₄-Na₂HPO₄ buffer (pH = 7.20, 0.1 M) with a flow rate of 20 mL/hour. The eluted fractions (4 mL in volume for each fraction) from the column were monitored by UV at 280 nm. Fractions #10-14 were collected to have 20 mL of immunogen (**36**). The concentration of immunogen was measured by using BCA Protein Concentration Assay. The Immunogen (**36**) has a concentration of 2.41 mg/mL with the hapten number of 925, and was used for the immunization of mice for antibody production.

### Preparation of compound (37)

To a solution of **25** (32 mg, 0.108 mmol) in THF (12 mL) was added NaH (19 mg, 0.752 mmol). The reaction mixture was stirred at room temperature for 10 minutes. 1,3-Dibromo-2-(methylsulfonyl)propane (43.8 mg, 0.156 mmol) was added to the reaction mixture, which was stirred at room temperature for 2 hours. Water (0.1 mL) was added and most of the THF was removed by rotary evaporation under reduced pressure. The residue was purified by flash column chromatography (silica gel) using ethyl acetate/hexane (1/2) as an eluent to give the desired product (**37**) (38 mg, 85.2 % yield); FAB-MS: MLi⁺ (420); ¹H-NMR (CDCl₃, 400 MHz) δ: 6.82 (m, 1H), 6.70 (m, 2H), 6.45, 6.36 (s, 1H), 6.16, 6.03 (s, 1H), 4.85 (s, 2H), 3.90 (m, 1H), 3.82 (s, 3H), 3.14 (s, 3H), 2.70 (m, 5H), 1.32 (m, 9H), 1.11 (bs, 3H).

### Preparation of compound (38)

To a solution of **37** (19 mg, 0.046 mmol) in CH₂Cl₂ (2 mL) was added TFA (0.3 mL). The reaction mixture was stirred at room temperature for 120 minutes. TLC analysis of the reaction showed that starting material (26) disappeared and a new, polar spot displayed (silica gel, ethyl acetate/hexane = 1/2). Most of CH₂Cl₂ and TFA were removed by rotary evaporation under reduced pressure. The residue was further dried under high vacuum to remove trace of TFA. This gave the desired product (**38**) (19 mg, 96% yield); ¹H-NMR (CDCl₃, 400 MHz) δ: 6.84 (m, 1H), 6.70 (m, 2H), 6.49 (s, 1H), 6.19 (s, 1H), 4.84 (s, 2H), 3.80 (s, 3H), 3.40 (m, 1H), 3.16 (s, 3H), 3.02 (m, 1H), 2.72 (m, 4H), 1.32 (d, *J* = 6.1 Hz, 3H).

### Preparation of compound (39)

To a solution of **25** (200 mg, 0.677 mmol) in DMF (15 mL) was added K₂CO₃ (280 mg, 2.03 mmol). The reaction mixture was stirred at room temperature for 15 minutes. Bromoacetonitrile (812 mg, 6.77 mmol) was added to the reaction mixture. The reaction mixture was heated at 80 °C for 18 hours. Most of the DMF was removed by rotary evaporation under reduced pressure and water (20 mL) was added. The aqueous phase was extracted with ethyl acetate (3 x 40 mL). The combined organic solvent was washed with water (20 mL) and dried over MgSO₄. The solvent was filtered and concentrated to dryness. The residue was purified by flash column chromatography (silica gel) using ethyl acetate/hexane (1/3) as an eluent to give the desired product (**39**) (204 mg, 90 % yield); ¹H-NMR (CDCl₃, 400 MHz) δ: 6.76 (m, 3H), 4.74 (s, 2H), 4.26 (m, 1H), 3.82 (s, 3H), 2.67 (m, 5H), 1.35 (m, 9H), 1.11 (m, 3H).

### Preparation of compound (40)

To a solution of **39** (50 mg, 0.15 mmol) in MeOH (8 mL) was added CoCl₂ 6H₂O (85 mg, 0.363 mmol). The reaction mixture was stirred at room temperature for 10 minutes. NaBH₄ (58 mg, 1.53 mmol) was added to the reaction mixture. The reaction mixture was stirred for 2 hours and then filtered. The black precipitate formed from the reaction was washed with CH₂Cl₂ (3 x 10 mL). The combined organic phases were concentrated to dryness. The residue was purified by flash column chromatography (silica gel) using MeOH/CH₂Cl₂ (1/4) as an eluent to give the desired product (**40**) (11 mg) with recovery of starting material, **39.** ¹H-NMR (CDCl₃, 400 MHz) δ: 6.78 (m, 3H), 5.28 (m, NH), 4.51, 4.24 (m, 1H), 4.00 (m, 2H), 3.83 (s, 3H), 3.07 (m, 2H), 2.69 (m, 5H), 1.35 (m, 9H), 1.12 (m, 3H).

### Assay using reagents in accordance with embodiments of the present invention

The antibodies and enzyme conjugates may be employed in assays for the detection of the respective analytes. Immunogen **10** (not part of this invention) is injected into sheep to raise antibody. The antibody obtained from the sheep bleed is spiked into the antibody diluent to prepare the antibody reagent. The antibody reagent consists of antibody as prepared above, buffer, stabilizers, preservatives, and the substrates for the enzyme conjugate NAD and glucose 6 phosphate.
Enzyme conjugate from compound **8** (not part of this invention) above is spiked into the conjugate reagent to prepare the enzyme conjugate reagent. The enzyme conjugate reagent consists of the conjugate, buffer, stabilizers and preservatives.

The antibody reagent and enzyme conjugate reagent are used in a homogeneous assay format to detect Ecstasy in urine samples. The analyzer (instrument) used to set up the assay is Syva 30-R Biochemical Analyzer (Syva Company, Cupertino CA). Ecstasy containing urine sample is incubated with antibody reagent followed by the addition of the enzyme conjugate reagent. The enzyme conjugate activity decreases upon binding to the antibody. The enzyme conjugate, which is not bound to the antibody, catalyzes the oxidation of glucose 6-phosphate (G6P). The oxidation of G6P is coupled with the reduction of NAD⁺ to NADH, which can be measured at 340 nm. The change in the absorbance at 340 nm can be measured spectrophotometrically. The Ecstasy concentration in a urine specimen can be measured in terms of G6PDH activity. The increase in the rate at 340 nm is due to the formation of NADH and is proportional to the enzyme conjugate activity. An assay curve is generated using MDMA spiked into negative urine. The assay rate increases with increasing the concentration of free drug in the sample.

Immunogens **30** and **36** were used to prepare antibodies according to methods similar to those described above.

## Claims

1. A compound of the formula: 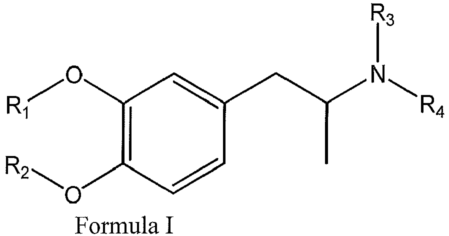 wherein:
R₁ is H, lower alkyl or a protecting group, wherein the term lower alkyl represents a branched or unbranched saturated monovalent hydrocarbon radical containing 1 to 10 carbon atoms, and wherein the protecting group is t-butoxycarbonyl (t-Boc), fluorenylmethyloxycarbonyl (Fmoc), acetaminomethyl (Acm), triphenyl methyl (Trt), benzyloxycarbonyl, biphenylisopropyloxycarbonyl, 1-amyloxycarbonyl, isobornyloxycarbonyl, alpha-dimethyl-3,5-dimethoxybenzyloxycarbonyl, o-nitrophenyl-sulfenyl, 2-cyano-1,1-dimethyl-ethoxycarbonyl bromobenzyloxy, carbamoyl, or formyl
R₂ is -(CH₂)ₙC(O)R₆ or -(CH₂)ₙR₆,
R₃ and R₄ are independently H or lower alkyl or a protecting group, wherein the term lower alkyl represents a branched or unbranched saturated monovalent hydrocarbon radical containing 1 to 10 carbon atoms, and wherein the protecting group is t-butoxycarbonyl (t-Boc), fluorenylmethyloxycarbonyl (Fmoc), acetaminomethyl (Acm), triphenyl methyl (Trt), benzyloxycarbonyl, biphenylisopropyloxycarbonyl, 1-amyloxycarbonyl, isobornyloxycarbonyl, alpha-dimethyl-3,5-dimethoxybenzyloxycarbonyl, o-nitrophenyl-sulfenyl, 2-cyano-1,1-dimethyl-ethoxycarbonyl bromobenzyloxy, carbamoyl, or formyl
R₆ is a label, wherein the term label stands for a catalyst, which is an enzyme, a chemiluminescent molecule, or a radioisotope, which may or may not be further labeled with a dye, a catalyst or another detectable group, and
n is an integer from 1 to 5,
and including acid salts thereof.

2. A compound according to Claim 1 wherein n is 1.

3. A method for determining a compound selected from the group consisting of 3,4-methylenedioxyamphetamine (MDA), 3,4-methylenedioxy-methamphetamine (MDMA), 3,4-methylenedioxyethylamphetamine (MDEA) and 4-hydroxy-3-methoxy-methamphetamine (HMMA), said method comprising:
(a) providing in combination in a medium:
(i) a sample suspected of containing said compound and
(ii) an antibody raised against a compound of the formula 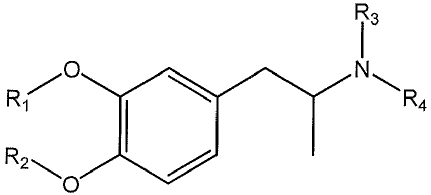 wherein:
R₁ is H, lower alkyl, or is taken together with R₂ to form a ring,
R₂ is H, lower alkyl, -(CH₂)ₙ₋C(O)R₆ or -(CH₂)n-R₆ or is taken together with R₁ to form a ring,
R₃ and R₄ are independently H or lower alkyl, or, when R₁ is taken together with R₂ to form a ring, at least one of R₃ and R₄ is -(CH₂)ₙ-C(O)R₅ or-(CH₂)ₙR₅, or, when R₁ is not taken together with R₂ to form a ring, at least one of R₁ and R₂ is not H or lower alkyl,
wherein in R₁ to R₄ the term lower alkyl represents a branched or unbranched saturated monovalent hydrocarbon radical containing 1 to 10 carbon atoms,
R₅ is an immunogenic carrier,
R₆ is an immunogenic carrier, and
n is an integer form 1 to 5
(iii) a label conjugate of the formula: 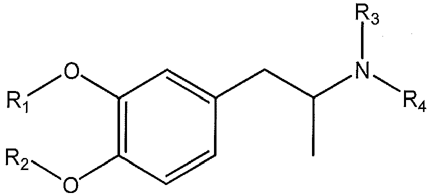 wherein:
R₁ is H, lower alkyl, wherein the term lower alkyl represents a branched or unbranched saturated monovalent hydrocarbon radical containing 1 to 10 carbon atoms,
R₂ is -(CH₂)ₙC(O)R₆ or -(CH₂)ₙR₆,
R₃ and R₄ are independently H or lower alkyl, wherein the term lower alkyl represents a branched or unbranched saturated monovalent hydrocarbon radical containing 1 to 10 carbon atoms,
R₆ is a label, wherein the term label stands for a catalyst, which is an enzyme, a chemiluminescent molecule, or a radioisotope, , which may or may not be further labeled with a dye, a catalyst or another detectable group, and
n is an integer from 1 to 5, and
(b) examining said medium for the presence of a complex comprising said compound and said antibody, the presence thereof indicating the presence of said compound in said sample, wherein
said examining comprises measuring signal from said label, the amount thereof being related to the presence of said compound in said sample, wherein said method is a homogeneous method and said medium is examined for the amount of said signal.

4. A method according to Claim 3 wherein n is 1.

5. A kit for determining a compound selected from the group consisting of 3,4-methylenedioxyamphetamine (MDA), 3,4-methylenedioxy-methamphetamine (MDMA), 3,4-methylenedioxyethylamphetamine (MDEA) and 4-hydroxy-3-methoxymethamphetamine (HMMA), said kit comprising:
(a) an antibody for said compound,
(b) a compound according to claim 1, which is a label conjugate of the formula: 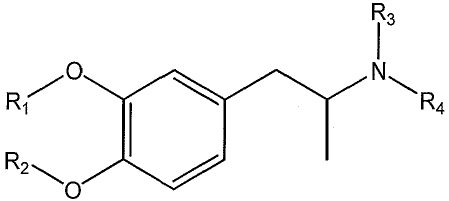 wherein:
R₁ is H or lower alkyl, wherein the term lower alkyl represents a branched or unbranched saturated monovalent hydrocarbon radical containing 1 to 10 carbon atoms,
R₂ is -(CH₂)ₙC(O)R₆ or -(CH₂)ₙR₆,
R₃ and R₄ are independently H or lower alkyl, wherein the term lower alkyl represents a branched or unbranched saturated monovalent hydrocarbon radical containing 1 to 10 carbon atoms,
R₆ is a label which is an enzyme, a chemiluminescent molecule or a radioisotope, and
n is an integer from 1 to 5, and
(c) ancillary reagents for determining said compound.

6. A method for determining methylenedioxyamphetamine and/or methylenedioxymethamphetamine and/or methylenedioxyethamphetamine in a sample suspected of containing methylenedioxyamphetamine and/or methylenedioxy-methamphetamine and/or methylenedioxyethamphetamine, said method comprising:
(a) providing in combination in a medium:
(i) said sample,
(ii) an antibody for methylenedioxyamphetamine, and/or
(iii) an antibody for methylenedioxymethamphetamine, and/or
(iv) an antibody for methylenedioxyethamphetamine, and
(ii) a compound according to claim 1 of the formula: 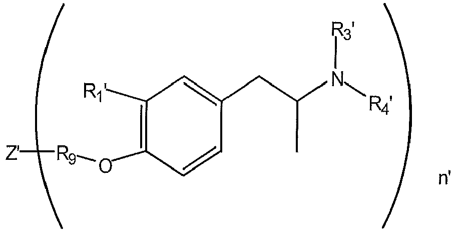 wherein:
R₁' is H, or methyl or ethyl
R₃' is H,
R₄' is H, or methyl or ethyl,
Rg is -(CH₂)ₙC(O)- or -(CH₂)-,
Z' which is an enzyme,
n is an integer from 1 to 5,
n' is an integer between 1 and the molecular weight of said enzyme divided by about 500; and
(b) examining said medium for the presence of a complex comprising said methylenedioxyamphetamine and said antibody for methylenedioxyamphetamine and/or a complex of said methylenedioxy-methamphetamine and said antibody for methylenedioxymethamphetamine and/or a complex of said methylenedioxyethamphetamine and said antibody for methylenedioxyethamphetamine, the presence thereof indicating the presence of said methylenedioxyamphetamine and/or methylenedioxy-methamphetamine and/or methylenedioxyethamphetamine in said sample.

## Patentansprüche

1. Verbindung der Formel: 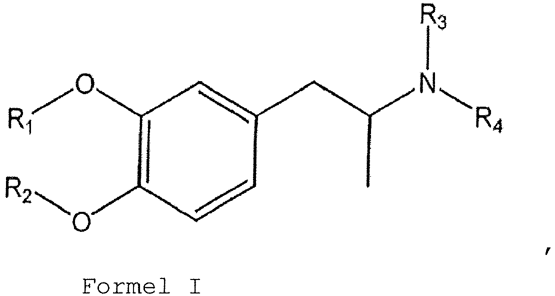 wobei:
R₁ für H, Niederalkyl oder eine Schutzgruppe steht, wobei der Begriff Niederalkyl für einen verzweigten oder geradkettigen gesättigten einwertigen Kohlenwasserstoffrest mit 1 bis 10 Kohlenstoffatomen steht und wobei es sich bei der Schutzgruppe um t-Butoxycarbonyl (t-Boc), Fluorenylmethyloxycarbonyl (Fmoc), Acetaminomethyl (Acm), Triphenylmethyl (Trt), Benzyloxycarbonyl, Biphenylisopropyloxycarbonyl, 1-Amyloxycarbonyl, Isobornyloxycarbonyl, alpha-Dimethyl-3,5-dimethoxybenzyloxycarbonyl, o-Nitrophenylsulfenyl, 2-Cyano-1,1-dimethylethoxycarbonyl, Brombenzyloxy, Carbamoyl oder Formyl handelt,
R₂ für -(CH₂)ₙC(O)R₆ oder -(CH₂)ₙR₆,
R₃ und R₄ unabhängig voneinander für H oder Niederalkyl oder eine Schutzgruppe stehen, wobei der Begriff Niederalkyl für einen verzweigten oder geradkettigen gesättigten einwertigen Kohlenwasserstoffrest mit 1 bis 10 Kohlenstoffatomen steht und wobei es sich bei der Schutzgruppe um t-Butoxycarbonyl (t-Boc), Fluorenylmethyloxycarbonyl (Fmoc), Acetaminomethyl (Acm), Triphenylmethyl (Trt), Benzyloxycarbonyl, Biphenylisopropyloxycarbonyl, 1-Amyloxycarbonyl, Isobornyloxycarbonyl, alpha-Dimethyl-3,5-dimethoxybenzyloxycarbonyl, o-Nitrophenylsulfenyl, 2-Cyano-1,1-dimethylethoxycarbonyl, Brombenzyloxy, Carbamoyl oder Formyl handelt,
R₆ für einen Marker steht, wobei der Begriff Marker für einen Katalysator, bei dem es sich um ein Enzym handelt, chemilumineszentes Molekül oder ein Radioisotop steht, der weiter mit einem Farbstoff, einem Katalysator oder einer anderen nachweisbaren Gruppe markiert sein kann oder nicht, und
n für eine ganze Zahl von 1 bis 5 steht,
einschließlich der Säuresalze davon.

2. Verbindung nach Anspruch 1, wobei n für 1 steht.

3. Verfahren zur Bestimmung einer Verbindung ausgewählt aus der Gruppe bestehend aus 3,4-Methylendioxyamphetamin (MDA), 3,4-Methylendioxymethamphetamin (MDMA), 3,4-Methylendioxyethylamphetamin (MDEA) und 4-Hydroxy-3-methoxymethamphetamin (HMMA), wobei das Verfahren Folgendes umfasst:
(a) die Bereitstellung, in Kombination in einem Medium,
(i) einer Probe, von der angenommen wird, dass sie die Verbindung enthält, und
(ii) eines Antikörpers gegen eine Verbindung der Formel 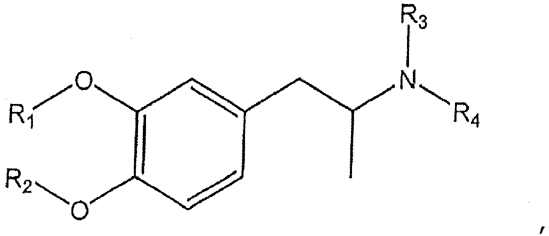 wobei:
R₁ für H oder Niederalkyl steht oder zusammen mit R₂ einen Ring bildet,
R₂ für H, Niederalkyl, -(CH₂)ₙ-C(O)R₆ oder -(CH₂)n-R₆ steht oder zusammen mit R₁ einen Ring bildet,
R₃ und R₄ unabhängig voneinander für H oder Niederalkyl stehen oder, wenn R₁ zusammen mit R₂ einen Ring bildet, mindestens einer der Reste R₃ und R₄ für -(CH₂)ₙ-C(O)R₅ oder -(CH₂)ₙR₅ steht, oder, wenn R₁ nicht zusammen mit R₂ einen Ring bildet, mindestens einer der Reste R₁ und R₂ nicht für H oder Niederalkyl steht,
wobei in R₁ bis R₄ der Begriff Niederalkyl für einen verzweigten oder geradkettigen gesättigten einwertigen Kohlenwasserstoffrest mit 1 bis 10 Kohlenstoffatomen steht,
R₅ für einen immunogenen Träger steht,
R₆ für einen immunogenen Träger steht und
n für eine ganze Zahl von 1 bis 5 steht,
(iii) eines Markerkonjugats der Formel: 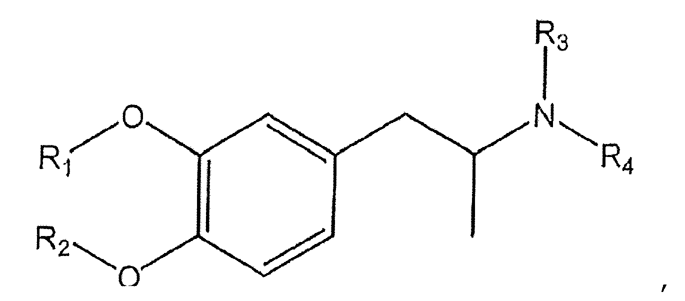 wobei:
R₁ für H oder Niederalkyl steht, wobei der Begriff Niederalkyl für einen verzweigten oder geradkettigen gesättigten einwertigen Kohlenwasserstoffrest mit 1 bis 10 Kohlenstoffatomen steht,
R₂ für -(CH₂)ₙC(O)R₆ oder -(CH₂)ₙR₆ steht,
R₃ und R₄ unabhängig voneinander für H oder Niederalkyl stehen, wobei der Begriff Niederalkyl für einen verzweigten oder geradkettigen gesättigten einwertigen Kohlenwasserstoffrest mit 1 bis 10 Kohlenstoffatomen steht,
R₆ für einen Marker steht, wobei der Begriff Marker für einen Katalysator, bei dem es sich um ein Enzym handelt, chemilumineszentes Molekül oder ein Radioisotop steht, der weiter mit einem Farbstoff, einem Katalysator oder einer anderen nachweisbaren Gruppe markiert sein kann oder nicht, und
n (b) für eine ganze Zahl von 1 bis 5 steht, und die Untersuchung des Mediums auf das Vorhandensein eines die Verbindung und den Antikörper umfassenden Komplexes, wobei dessen Anwesenheit auf das Vorhandensein der Verbindung in der Probe deutet, wobei
das Untersuchen das Messen eines Signals von dem Marker umfasst, wobei dessen Menge mit dem Vorhandensein der Verbindung in der Probe in Zusammenhang steht, wobei es sich bei dem Verfahren um ein homogenes Verfahren handelt und das Medium auf die Menge des Signals untersucht wird.

4. Verfahren nach Anspruch 3, wobei n für 1 steht.

5. Kit zur Bestimmung einer Verbindung ausgewählt aus der Gruppe bestehend aus 3,4-Methylendioxyamphetamin (MDA), 3,4-Methylendioxymethamphetamin (MDMA), 3,4-Methylendioxyethylamphetamin (MDEA) und 4-Hydroxy-3-methoxymethamphetamin (HMMA), wobei das Kit Folgendes umfasst:
(a) einen Antikörper gegen die Verbindung,
(b) eine Verbindung nach Anspruch 1, bei der es sich um ein Markerkonjugat der Formel: 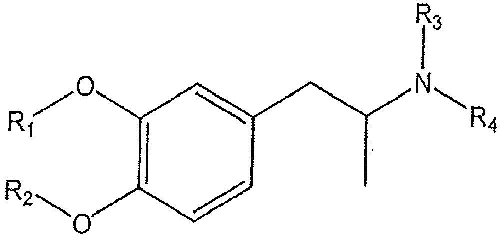 handelt, wobei:
R₁ für H oder Niederalkyl steht, wobei der Begriff Niederalkyl für einen verzweigten oder geradkettigen gesättigten einwertigen Kohlenwasserstoffrest mit 1 bis 10 Kohlenstoffatomen steht,
R₂ für -(CH₂)ₙC(O)R₆ oder -(CH₂)ₙR₆ steht,
R₃ und R₄ unabhängig voneinander für H oder Niederalkyl stehen, wobei der Begriff Niederalkyl für einen verzweigten oder geradkettigen gesättigten einwertigen Kohlenwasserstoffrest mit 1 bis 10 Kohlenstoffatomen steht,
R₆ für einen Marker steht, bei dem es sich um ein Enzym, ein chemilumeszentes Molekül oder einen Radioisotopen handelt, und
n für eine ganze Zahl von 1 bis 5 steht, und
(c) Hilfsreagenzien zur Bestimmung der Verbindung.

6. Verfahren zur Bestimmung von Methylendioxyamphetamin und/oder Methylendioxymethamphetamin und/oder Methylendioxyethamphetamin in einer Probe, von der angenommen wird, dass sie Methylendioxyamphetamin und/oder Methylendioxymethamphetamin und/oder Methylendioxyethamphetamin enthält, wobei das Verfahren Folgendes umfasst:
(a) die Bereitstellung, in Kombination in einem Medium,
(i) der Probe,
(ii) eines Antikörpers gegen Methylendioxyamphetamin und/oder
(iii) eines Antikörpers gegen Methylendioxymethamphetamin und/oder
(iv) eines Antikörpers gegen Methylendioxyethamphetamin und
(ii) einer Verbindung gemäß Anspruch 1 mit der Formel: 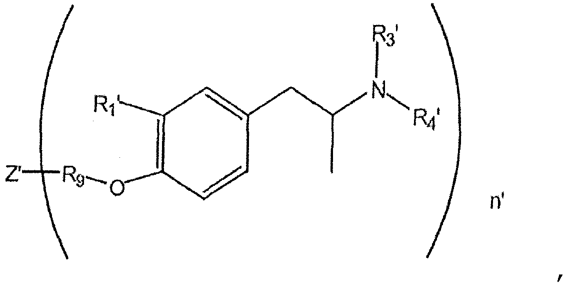 wobei:
R₁' für H, Methyl oder Ethyl steht,
R₃' für H steht,
R₄' für H, Methyl oder Ethyl steht,
R₉ für -(CH₂)ₙC(O)- oder -(CH₂)- steht,
Z' für ein Enzym steht,
n für eine ganze Zahl von 1 bis 5 steht
n' für eine ganze Zahl zwischen 1 und dem Molekulargewicht des Enzyms geteilt durch etwa 500 steht, und
(b) das Untersuchen des Mediums auf das Vorhandensein eines das Methylendioxyamphetamin und den Antikörper gegen Methylendioxyamphetamin umfassenden Komplexes und/oder eines Komplexes des Methylendioxymethamphetamins und des Antikörpers gegen Methylendioxymethamphetamin und/oder eines Komplexes des Methylendioxyethamphetamins und des Antikörpers gegen Methylendioxyethamphetamin, wobei dessen Anwesenheit auf das Vorhandensein von Methylendioxyamphetamin und/oder Methylendioxymethamphetamins und/oder Methylendioxyethamphetamins in der Probe deutet.

## Revendications

1. Composé de formule : 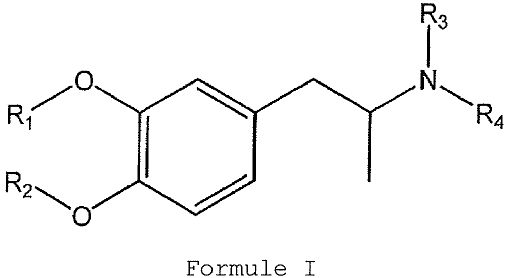 dans lequel :
R₁ est H, un alkyle inférieur ou un groupe protecteur, dans lequel le terme alkyle inférieur représente un radical hydrocarbure monovalent saturé, ramifié ou non ramifié, contenant de 1 à 10 atomes de carbone, et dans lequel le groupe protecteur est le t-butoxycarbonyle (t-Boc), le fluorénylméthyloxycarbonyle (Fmoc), l'acétaminométhyle (Acm), le triphénylméthyle (Trt), le benzyloxycarbonyle, le biphénylisopropyloxycarbonyle, le 1-amyloxycarbonyle, l'isobornyloxycarbonyle, l'alpha-diméthyl-3,5-diméthoxybenzyloxycarbonyle, l'o-nitrophényl-sulfényle, le 2-cyano-1,1-dimethyl-éthoxycarbonyle, le bromobenzyloxy, le carbamoyle ou le formyle
R₂ est -(CH₂)ₙC(O)R₆ ou -(CH₂)ₙR₆,
R₃ et R₄ sont indépendamment H ou un alkyle inférieur ou un groupe protecteur, dans lequel le terme alkyle inférieur représente un radical hydrocarbure monovalent saturé, ramifié ou non ramifié, contenant de 1 à 10 atomes de carbone, et dans lequel le groupe protecteur est le t-butoxycarbonyle (t-Boc), le fluorénylméthyloxycarbonyle (Fmoc), l'acétaminométhyle (Acm), le triphénylméthyle (Trt), le benzyloxycarbonyle, le biphénylisopropyloxycarbonyle, le 1-amyloxycarbonyle, l'isobornyloxycarbonyle, l'alpha-diméthyl-3,5-diméthoxybenzyloxycarbonyle, l'o-nitrophényl-sulfényle, le 2-cyano-1,1-dimethyl-éthoxycarbonyle, le bromobenzyloxy, le carbamoyle ou le formyle
R₆ est un marqueur, dans lequel le terme marqueur désigne un catalyseur, qui est une enzyme, une molécule chimioluminescente ou un radio-isotope, qui peut ou peut ne pas être en outre marqué par un colorant, un catalyseur ou un autre groupe détectable, et
n est un nombre entier de 1 à 5,
et comprenant les sels d'acide de celui-ci.

2. Composé selon la revendication 1 dans lequel n vaut 1.

3. Procédé de détermination d'un composé choisi dans le groupe constitué de la 3,4-méthylènedioxyamphétamine (MDA), de la 3,4-méthylènedioxy-méthamphétamine (MDMA), de la 3,4-méthylènedioxyéthylamphétamine (MDEA) et de la 4-hydroxy-3-méthoxy-méthamphétamine (HMMA), ledit procédé comprenant :
(a) la fourniture en association dans un milieu :
(i) d'un échantillon soupçonné de contenir ledit composé et
(ii) d'un anticorps dirigé contre un composé de formule 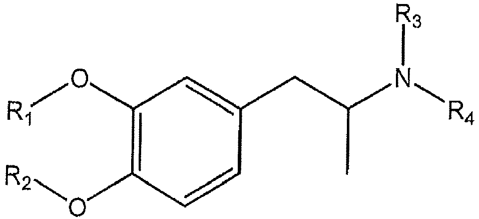 dans lequel :
R₁ est H, un alkyle inférieur, ou est considéré conjointement avec R₂ pour former un cycle,
R₂ est H, un alkyle inférieur, -(CH₂)ₙ-C(O)R₆ ou -(CH₂)ₙ-R₆ ou est considéré conjointement avec R₁ pour former uncycle,
R₃ et R₄ sont indépendamment H ou un alkyle inférieur, ou, lorsque R₁ est considéré conjointement avec R₂ pour former un cycle, au moins l'un de R₃ et R₄ est -(CH₂)ₙ-C(O)R₅ ou -(CH₂)ₙR₅, ou, lorsque R₁ n'est pas considéré conjointement avec R₂ pour former un cycle, au moins l'un de R₁ et R₂ n'est pas H ou un alkyle inférieur, dans lequel dans R₁ à R₄ le terme alkyle inférieur représente un radical hydrocarbure monovalent saturé, ramifié ou non ramifié, contenant de 1 à 10 atomes de carbone,
R₅ est un vecteur immunogène,
R₆ est un vecteur immunogène, et
n est un nombre entier de 1 à 5
(iii) d'un conjugué marqueur de la formule : 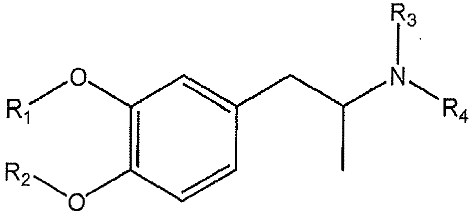 dans lequel :
R₁ est H, un alkyle inférieur, dans lequel le terme alkyle inférieur représente un radical hydrocarbure monovalent saturé, ramifié ou non ramifié, contenant de 1 à 10 atomes de carbone,
R₂ est -(CH₂)ₙC(O)R₆ ou -(CH₂)ₙR₆,
R₃ et R₄ sont indépendamment H ou un alkyle inférieur,
dans lequel le terme alkyle inférieur représente un radical hydrocarbure monovalent saturé, ramifié ou non ramifié, contenant de 1 à 10 atomes de carbone,
R₆ est un marqueur, dans lequel le terme marqueur désigne un catalyseur, qui est une enzyme, une molécule chimioluminescente ou un radio-isotope, qui peut ou
peut ne pas être en outre marqué par un colorant, un catalyseur ou un autre groupe détectable, et
n est un nombre entier de 1 à 5, et
(b) l'examen dudit milieu pour déceler la présence d'un complexe comprenant ledit composé et ledit anticorps, la présence de celui-ci indiquant la présence dudit composé dans ledit échantillon, dans lequel ledit examen comprend la mesure du signal provenant dudit marqueur, la quantité de celui-ci étant liée à la présence dudit composé dans ledit échantillon, dans lequel ledit procédé est un procédé homogène et ledit milieu est examiné pour déterminer la quantité dudit signal.

4. Procédé selon la revendication 3 dans lequel n vaut 1.

5. Nécessaire pour déterminer un composé choisi dans le groupe constitué de la 3,4-méthylènedioxyamphétamine (MDA), de la 3,4-méthylènedioxy-méthamphétamine (MDMA), de la 3,4-méthylènedioxyéthylamphétamine (MDEA) et de la 4-hydroxy-3-méthoxyméthamphétamine (HMMA), ledit nécessaire comprenant :
(a) un anticorps pour ledit composé,
(b) un composé selon la revendication 1, qui est un conjugué marqueur de formule : 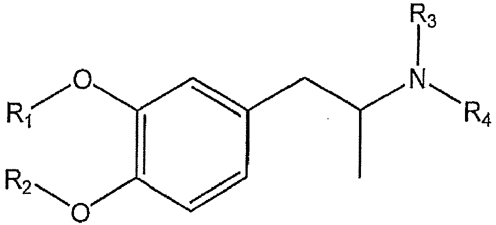 dans lequel :
R₁ est H ou un alkyle inférieur, dans lequel le terme alkyle inférieur représente un radical hydrocarbure monovalent saturé, ramifié ou non ramifié, contenant de 1 à 10 atomes de carbone,
R₂ est -(CH₂)ₙC(O)R₆ ou -(CH₂)ₙR₆,
R₃ et R₄ sont indépendamment H ou un alkyle inférieur,
dans lequel le terme alkyle inférieur représente un radical hydrocarbure monovalent saturé, ramifié ou non ramifié, contenant de 1 à 10 atomes de carbone,
R₆ est un marqueur qui est une enzyme, une molécule chimioluminescente ou un radio-isotope, et
n est un nombre entier de 1 à 5, et
(c) des réactifs auxiliaires pour déterminer ledit composé.

6. Procédé de détermination de la méthylènedioxyamphétamine et/ou de la méthylènedioxyméthamphétamine et/ou de la méthylènedioxyéthamphétamine dans un échantillon soupçonné de contenir de la méthylènedioxyamphétamine et/ou de la méthylènedioxy-méthamphétamine et/ou de la méthylènedioxyéthamphétamine, ledit procédé comprenant :
(a) la fourniture en association dans un milieu :
(i) dudit échantillon,
(ii) d'un anticorps pour la méthylènedioxyamphétamine, et/ou
(iii) d'un anticorps pour la méthylènedioxyméthamphétamine, et/ou
(iv) d'un anticorps pour la méthylènedioxyéthamphétamine, et
(ii) d'un composé selon la revendication 1 de formule : 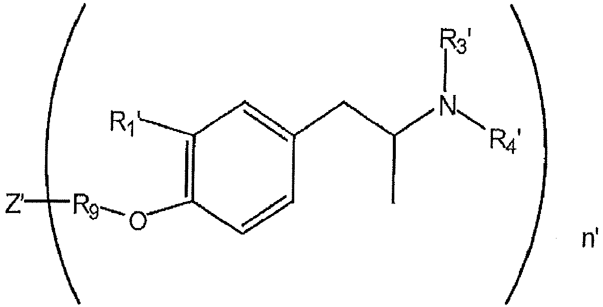 dans lequel :
R₁' est H, ou un méthyle ou un éthyle
R₃' est H,
R₄' est H, ou un méthyle ou un éthyle,
Rg est -(CH₂)ₙC(O)- ou -(CH₂)-,
Z' qui est une enzyme,
n est un nombre entier de 1 à 5,
n' est un nombre entier entre 1 et le poids moléculaire de ladite enzyme divisé par environ 500 ; et
(b) l'examen dudit milieu pour déceler la présence d'un complexe comprenant ladite méthylènedioxyamphétamine et ledit anticorps pour la méthylènedioxyamphétamine et/ou d'un complexe de ladite méthylènedioxy-méthamphétamine et dudit anticorps pour la méthylènedioxyméthamphétamine et/ou d'un complexe de ladite méthylènedioxyéthamphétamine et dudit anticorps pour la méthylènedioxyéthamphétamine, la présence de celui-ci indiquant la présence de ladite méthylènedioxyamphétamine et/ou de ladite méthylènedioxy-méthamphétamine et/ou de ladite méthylènedioxyéthamphétamine dans ledit échantillon.
